(19) Europäisches Patentamt
European Patent Office
Office européen des brevets

(11) EP 0 428 902 B1

(12) **EUROPEAN PATENT SPECIFICATION**

(45) Date of publication and mention of the grant of the patent:
**05.06.1996 Bulletin 1996/23**

(51) Int Cl.[6]: **G03C 7/38**

(21) Application number: **90120451.1**

(22) Date of filing: **25.10.1990**

# (54) Silver halide color photographic materials

Farbfotografische Silberhalogenidmaterialien

Matériaux photographiques couleur à l'halogénure d'argent

(84) Designated Contracting States:
**DE FR GB NL**

(30) Priority: **25.10.1989 JP 278016/89**

(43) Date of publication of application:
**29.05.1991 Bulletin 1991/22**

(73) Proprietor: **FUJI PHOTO FILM CO., LTD.**
**Kanagawa (JP)**

(72) Inventors:
- **Sato, Tadahisa**
**Minami Ashigara-shi, Kanagawa (JP)**
- **Mizukawa, Yuki**
**Minami Ashigara-shi, Kanagawa (JP)**
- **Naruse, Hideaki**
**Minami Ashigara-shi, Kanagawa (JP)**

(74) Representative: **Patentanwälte Dr. Solf & Zapf**
**Candidplatz 15**
**81543 München (DE)**

(56) References cited:
**EP-A- 0 177 765**
**EP-A- 0 219 033**
**EP-A- 0 326 406**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 428 902 B1

## Description

### FIELD OF THE INVENTION

The present invention relates to silver halide color photographic materials and, more particularly, describes couplers which provide for more adaptable color photographic systems in which silver halide color photographic materials using such couplers are improved with respect to processing dependence, that is, they exhibit greater tolerance to processing condition variations. Even more particularly, the present invention relates to silver halide color photographic materials which contain 1H-pyrazolo[1,5-b][1,2,4]triazole couplers which have aryloxy groups as coupling split-off groups which enable the processing dependence to be improved.

### BACKGROUND OF THE INVENTION

Many methods exist in which photosensitive silver halide emulsions are employed with so-called dye forming couplers (referred to hereinafter simply as couplers) which react with the oxidation products of primary aromatic amine developing agents after exposure of the silver halide to form dyes of a wide variety of colors in silver halide color photographic materials. Further, the combined use in a photosensitive material of yellow couplers, cyan couplers and magenta couplers which are incorporated into the emulsions of different layers is conventionally known in the photographic arts.

Among these couplers, 5-pyrazolone based couplers and pyrazolotriazole-based couplers are used in conventional practice as magenta couplers. The pyrazolotriazole-based coupler is of particular importance in that the azomethine dyes formed therefrom do not have undesirable side absorbance in the region of 430 nm which is observed with the azomethine dyes formed from the 5-pyrazolone-based coupler. Additionally, the pyrazolotriazole-based couplers have a good cut-off on the long wavelength side of the absorption spectrum so that they provide good color reproduction. There are two known groups of pyrazolotriazole couplers having different chemical structures. One group comprises the 1H-pyrazolo[5,1-c][1,2,4]triazoles as disclosed in US-A- 3,725,067, and the other group are the 1H-pyrazolo[1,5-b][1,2,4]triazoles disclosed in JP-A-59-171956 and US-A- 4,540,654. (The term "JP-A" as used herein means an "unexamined published Japanese patent application".)

Because of the current wide spread use of silver halide color photography, development processing is no longer performed by only a very limited number of development facilities and, instead, processing is increasingly being carried out in a more disseminated manner as evidenced by the growth of mini-laboratory operations, for example. Moreover, at the same time, strong demands for more rapid and simpler processing have arisen as another trend in photographic arts. As a result, there are many likely instances where processing will be carried out in conditions where it is difficult to control the processing bath composition and where color photographic films and printing papers will be processed in development baths containing solutions which are subject to wide variations in overall composition. Against this background, the design of photosensitive materials which are less likely to be affected by processing fluctuations than in the past has become very desirable.

As mentioned above, the 1H-pyrazolo[1,5-b][1,2,4]triazole couplers, which are one type of conventionally known pyrazolotriazole-based couplers, have excellent hues and the use of these couplers greatly improves the color reproduction in a color photograph. However, since the coupling activity of these pyrazolotriazole couplers with the oxidized forms of developing agents is lower than that of the 5-pyrazolone-based couplers, for example, these couplers have a disadvantage in that they are highly processing dependent. With the current trends in the color photography field as described above, it is clear that improvement is needed in this respect.

In the past, methods involving coupling split-off groups, methods involving substituent groups in the 2- and 6-positions and methods involving ballast groups have been advanced as means of improving these pyrazolotriazole couplers. Among these methods, the methods involving a coupling split-off group are useful in that the coupling activity can be controlled without altering the hue of the azomethine dye which is formed, and in the past halogen atom split-off types, oxygen atom split-off types, nitrogen atom split-off types and sulfur atom split-off types have been proposed as useful coupling split-off groups. Aryloxy split-off groups are one noteworthy example of the oxygen atom split-off types of the various types of coupling split-off groups indicated above and represent an excellent means of increasing the coupling activity as has been disclosed in JP-A-61-53644. However, the actual performance of such aryloxy compounds disclosed in this published Japanese patent application is insufficient to meet the current requirements in respect of rapid processing in variable processing conditions.

EP-A-0 177 765 discloses a silver halide color photographic material comprising a support having thereon at least one silver halide emulsion layer, in which said silver halide emulsion layer or a layer adjacent thereto contains a pyrazoloazole magenta coupler having at least one substituted alkyl group represented by the formula (I):

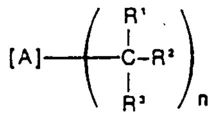

(I)

wherein (A) represents a pyrazoloazole magenta coupler residual group; $R^1$ is an alkyl group; $R^2$ and $R^3$ each is a hydrogen atom or a substituent , provided that both $R^2$ and $R^3$ are not hydrogen atoms at the same time; and n is 1, 2 or 3.

EP-A- 0 219 033 describes a silver halide color photographic light-sensitive material comprising at least one silver halide light-sensitive emulsion layer associated with a magenta coupler provided on a support, wherein at least one of said silver halide emulsion layer and the layer(s) adjacent to the emulsion layer contains a hydroquinone derivative, wherein said coupler is at least one compound selected from the group consisting of compounds represented by formula (I), biscompounds derived from the compounds, and polymers having coupler residues derived from the compounds,

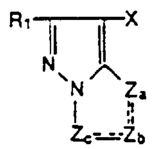

(I)

wherein $R_1$ represents a hydrogen atom or a substituent; X represents a hydrogen atom or group which can be released therefrom upon coupling with an oxidized form of an aromatic primary amine-developing agent; and Za, Zb, and Zc each represents a methine, substituted methine, =N-, or -NH-, one of Za-Zb bond and Zb-Zc bond is a double bond and the other is a single bond, and when the Zb-Zc bond is a carbon-carbon double bond, it can form a part of an aromatic ring, or $R_1$ or X is a group forming the bis-compound or the polymer or Za, Zb, or Zc represents a substituted methine forming the bis-compound or the polymer, and said hydroquinone derivative is represented by formula (II):

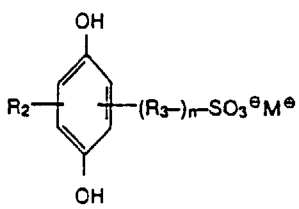

(II)

wherein $R_2$ represents a substituted or unsubstituted alkyl group, alkoxy group, aromatic group, or alkylthio group; $R_3$ represents an alkylene group; n represents an integer of 0 or 1; and M represents a cation.

EP-A-0 326 406 discloses a silver halide photographic light-sensitive material which has excellent characteristics peculiar to pyrazoloazole type magenta couplers and yet good suitability to rapid processing, and shows improvement in the gradiation in respect of softening at the toe portion of characteristic curve thereof without impairment of the image preservability and other photographic properties. The photographic material comprises a support having thereon a silver halide emulsion layer containing a silver halide grain having a silver chloride content of not less than 90 mole%, a compound represented by the following formula I, and a compound capable of deactivating the oxidized product of a color developing agent with a relative reaction rate of not less than 1:6;

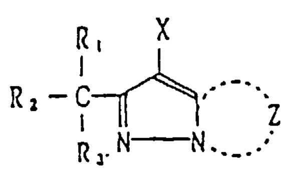

Formula I

wherein $R_1$, $R_2$ and $R_3$ each represent a substituent other than hydrogen atom, which may be the same as or different from each other; Z represents a group of non-metal atoms necessary for completing a hetereocyclic ring which may have a substituent; X represents a hydrogen atom or a group capable of being split off upon reaction with the oxidized product of a color developing agent.

The present inventors have carried out investigations in orders to determine whether or not there are couplers of the phenoxy split-off type which are distinguished by having both a high coupling activity and which have a small

processing dependence. Further, from a practical point of view, the maximum absorption peak wavelength λ of the dye which is obtained by coupling of the coupler with an oxidized color developer should be within the preferred range of 540 to 545 nm in the film, and the coupler should be one which can be prepared easily.

Accordingly, an object of the invention is to provide aryloxy split-off type pyrazolo[1,5-b]-1,2,4-triazole couplers which have an excellent processing adaptability such that they can be used in the state of the art rapid and simplified processing operations (which have limited controls on processing conditions) and which have an excellent hue and preparative ease.

Another object of the present invention is to provide silver halide color photographic materials in which these couplers are used.

As a result of thorough and extensive investigation, the present inventors have discovered that the above mentioned objects can be realized with a group of a certain type of aryloxy split-off type couplers. That is to say the above-mentioned objects have been realized by means of a silver halide color photographic material comprising a support having thereon at least one silver halide emulsion layer containing at least one coupler which is represented by formula (II) as follows:

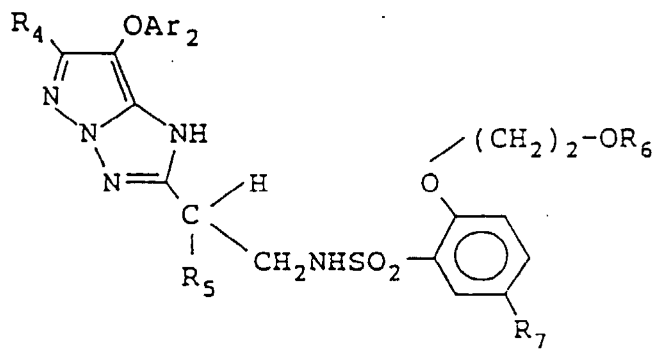

(II)

wherein $R_4$ and $R_5$ each represents an alkyl group; $R_6$ represents an alkyl group or an aryl group; $R_7$ represents a substituent group; and $Ar_2$ represents an aryl group.

## DETAILED DESCRIPTION OF THE INVENTION

The substituent groups in (II) are described in detail below.

$R_4$ and $R_5$ each represents an alkyl group, and more precisely each represents a linear chain or branched chain alkyl group which has from 1 to 35 carbon atoms. Even more particularly, each represents an alkyl group, for example, a methyl, ethyl, isopropyl, tertbutyl, octyl, butyl, 1-ethylpenthyl or cyclohexyl group. $R_4$ and $R_5$ are preferably a methyl, ethyl, isopropyl or tertbutyl group, and even more preferably $R_4$ is an ethyl or isopropyl group and $R_5$ is a methyl group.

$R_6$ represents an alkyl group or an aryl group, and more particularly $R_6$ is a substituted or unsubstituted, linear chain or branched chain alkyl group which has at least 2 carbon atoms or a substituted or unsubstituted aryl group which has at least 6 carbon atoms.

$R_6$ is an alkyl group such as, for example, methyl, ethyl, 2-ethylhexyl, butyl, hexyl, octyl, octyl-oxyethyl or phenoxyethyl, or an aryl group such as, for example, phenyl, 4-tert-butylphenyl, 2,4-di-tert-butylphenyl, 4-tert-octylphenyl, 2,4-di-tertoctylphenyl, 4-methoxyphenyl or 2-naphthyl.

Preferably $R_6$ is an alkyl group. More preferably, $R_6$ is an ethyl, butyl, hexyl or octyl group.

Most preferably, $R_6$ is an ethyl, hexyl or octyl group.

The substituent groups which may be present on $R_6$ include substituent groups such as, for example, a halogen atom, an alkyl group, an aryl group, a heterocyclic group, a cyano group, a hydroxyl group, a nitro group, a carboxyl group, a sulfo group, an amino group, an alkoxy group, an aryloxy group, an acylamino group, an alkylamino group, an anilino group, a ureido group, a sulfamoylamino group, an alkylthio group, an arylthio group, an alkoxycarbonylamino group, a sulfonamido group, a carbamoyl group, a sulfamoyl group, a sulfonyl group, an alkoxycarbonyl group, a heterocyclic oxy group, an azo group, an acyloxy group, a carbamoyloxy group, a silyloxy group, an aryloxycarbonylamino group, an imido qroup, a heterocyclic thio group, sulfinyl group, a phosphonyl group, an aryloxycarbonyl group, an acyl group and an azolyl group.

$R_7$ represents a substituent group, and more particularly it is a substituent group of the same types described above for $R_6$. $R_7$ is preferably an alkyl group, an alkoxy group, an acylamino group or a sulfonamido group, and more particularly these preferred groups are a linear chain or branched chain alkyl group which has at least 4 carbon atoms, an alkoxy group which has at least 4 carbon atoms, an acylamino group which has at least 5 carbon atoms or an alkylsulfonamido or arylsulfonamido group which has at least 6 carbon atoms.

More desirably, $R_7$ is a branched chain alkyl group which has at least 4 carbon atoms, an alkoxyl group which has

at least 8 carbon atoms, an acyl amino group which as at least 5 carbon atoms or a substituted benzenesulfonamido group which has at least 18 carbon atoms, and most preferably, $R_7$ is a tert-butyl group or a 2-alkoxy-5-alkylbenzenesulfonamido group which has at least 18 carbon atoms.

$Ar_2$ represents an aryl group, and more precisely $Ar_2$ represents a phenyl group or a naphthyl group, and these may have the same substituent groups as those described heretofore as substituent groups for $R_6$. More precisely, $Ar_2$ is, for example, a substituted phenyl group or naphthyl group such as 2-, 3- or 4-alkylphenyl, 2-, 3- or 4-carboxyphenyl, 2-, 3- or 4-alloxyphenyl, 2-, 3- or 4-alkoxycarbonyl, 2-, 3- or 4-acylaminophenyl, 2-, 3--or 4-alkylsulfonamidophenyl, 2-, 3- or 4-benzenesulfonamidophenyl, 2-, 3- or 4-alkylsulfonylphenyl, 2-, 3- or 4-benzenesulfonylphenyl or 4-alkyl-1-naphthyl. $Ar_1$ or $Ar_2$ are preferably a 4-alkylphenyl, 2-, 3- or 4-carboxyphenyl or 4-benzenesulfonylphenyl group; more preferably a 4-alkylphenyl group; and most preferably a 4-methylphenyl group.

A bis-norm may be formed by, $R_4$, $R_5$, $R_6$, $R_7$, Ar, or $Ar_2$ becoming a divalent group.

In instances where the units represented by formula (II) are included in a vinyl monomer, the vinyl group may have substituent groups other than those indicated for formula (II), and the preferred substituent groups for the vinyl group are a hydrogen atom, chlorine atom or a lower alkyl group which has from 1 to 4 carbon atoms (for example, methyl, ethyl).

The monomers which contain units which can be represented by formula (II) can be formed into copolymers with non-color forming ethylenic monomers which do not undergo a coupling reaction with the oxidation products of primary aromatic amine developing agents.

Examples of non-color forming ethylenic monomers which do not undergo a coupling reaction with the oxidation products of a primary aromatic amine developing agent include acrylic acid, $\alpha$-chloroacrylic acid, $\alpha$-alkylacrylic acids (for example, methacrylic acid) and esters and amides derived from acrylic acids, (for example, acrylamide, n-butylacrylamide, tert-butylacrylamide, diacetoneacrylamide, methacrylamide, methyl acrylate, ethyl acrylate, n-propyl acrylate, n-butyl acrylate, tert-butyl acrylate, isobutyl acrylate, 2-ethylhexyl acrylate, n-octyl acrylate, lauryl acrylate, methyl methacrylate, ethyl methacrylate, n-butyl -methacrylate and $\beta$-hydroxyethyl methacrylate), methylene di-bisacrylamide, vinyl esters (for example, vinyl acetate, vinyl propionate and vinyl laurate), acrylonitrile, methacrylonitrile, aromatic vinyl compounds (for example, styrene and derivatives thereof, vinyltoluene, divinylbenzene, vinylacetophenone and sulfostyrene), itaconic acid, citraconic acid, crotonic acid, vinylidene chloride, vinyl alkyl ethers (for example, vinyl ethyl ether), maleic acid, maleic anhydride, maleic acid esters, N-vinyl-2-pyrrolidone, N-vinylpyridine and 2- and 4-vinylpyridines. Two or more of the non-color forming ethylenically unsaturated monomers described herein can be used conjointly. For example, such co-joint usage can be made of n-butylacrylamide and methyl acrylate, styrene and methacrylic acid, methacrylic acid and acrylamide, or methyl methacrylate and diacetoneacrylamide.

As is well known in the polymer color coupler field, the non-color forming ethylenically unsaturated monomer which is used for copolymerization with a solid water insoluble monomeric coupler can be selected in such a way as to have a beneficial effect on the physical and/or chemical properties of the coupler, such as the solubility, compatibility with binders such as gelatin which are used in photographic colloid compositions, flexibility and thermal stability, and the like, of the copolymer which is formed.

The polymer couplers used in the present invention may be water soluble or water insoluble, and from among these materials the polymer coupler latexes are especially desirable.

Specific examples of couplers which can be represented by formulae (II) of the present invention are indicated below, but the present invention is not to be construed as limited by these examples.

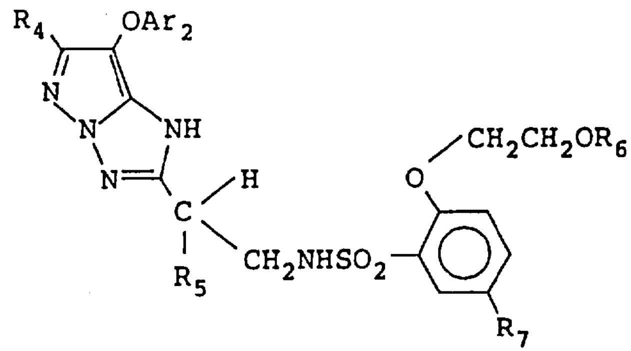

(II)

| | $R_4$ | $R_5$ | $R_6$ | $R_7$ | $Ar_2$ |
|---|---|---|---|---|---|
| m-1 | $CH_3$ | $CH_3$ | $C_6H_{13}$ | t-$C_8H_{17}$ | 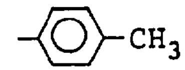 |
| m-2 | $C_2H_5$ | " | " | " | " |
| m-3 | i-$C_3H_7$ | " | " | " | " |
| m-4 | t-$C_4H_9$ | " | " | " | " |
| m-5 | $C_2H_5$ | " | $C_8H_{17}$ | " | " |
| m-6 | $C_2H_5$ | $C_2H_5$ | $C_6H_{13}$ | " | 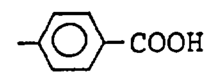 |
| m-7 | i-$C_3H_7$ | $CH_3$ | " | " | " |
| m-8 | t-$C_4H_9$ | " | " | " | " |

| | $R_4$ | $R_5$ | $R_6$ | $R_7$ | $Ar_2$ |
|---|---|---|---|---|---|
| m-9 | $t-C_4H_9$ | $CH_3$ | $C_2H_5$ | 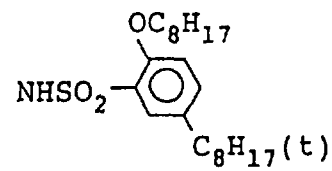 | 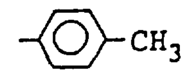 |
| m-10 | $i-C_3H_7$ | " | $C_2H_5$ | 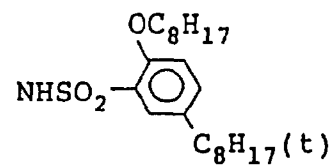 | 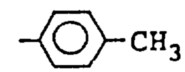 |
| m-11 | $C_2H_5$ | " | " | " | " |
| m-12 | " | $C_2H_5$ | $C_6H_{17}$ | 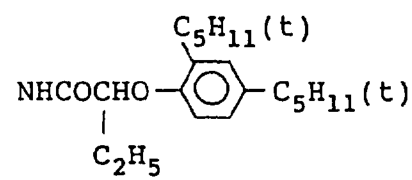 | 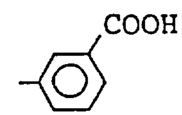 |
| m-13 | $i-C_3H_7$ | $i-C_3H_7$ | " | $NHCOC(CH_3)_3$ | 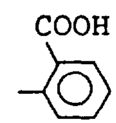 |
| m-14 | t-Bu | $CH_3$ | $-C_2H_4OC_2H_5$ | $t-C_8H_{17}$ | 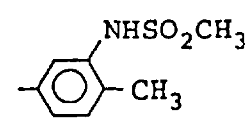 |

| | $R_4$ | $R_5$ | $R_6$ | $R_7$ | $Ar_2$ |
|---|---|---|---|---|---|
| m-15 | t-Bu | $CH_3$ | 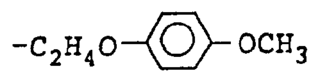 | $t\text{-}C_8H_{17}$ | 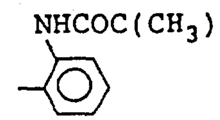 |
| m-16 | " | " | $-C_2H_4OCH_2CH_2OC_6H_{13}$ | " | 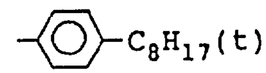 |
| m-17 | $i\text{-}C_3H_7$ | " | $-C_6H_{13}$ | " | 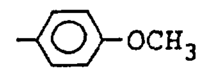 |
| m-18 | " | " | " | " | 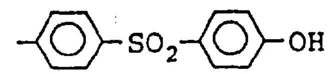 |
| m-19 | " | " | " | " | 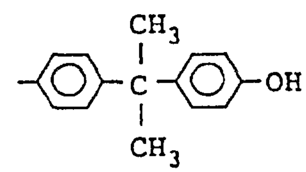 |
| m-20 | " | " | " | " | 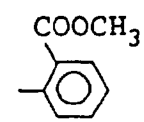 |
| m-21 | " | " | " | " | 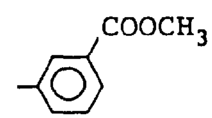 |

| | $R_4$ | $R_5$ | $R_6$ | $R_7$ | $Ar_2$ |
|---|---|---|---|---|---|
| m-22 | $i-C_3H_7$ | $CH_3$ | $-C_6H_{13}$ | $t-C_8H_{17}$ | 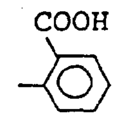 |
| m-23 | t-Bu | " | " | " | 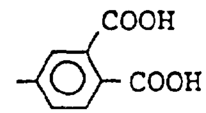 |
| m-24 | " | " | " | " | 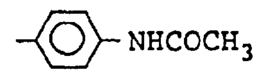 |
| m-25 | $C_2H_5$ | " | " | " | 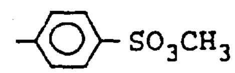 |
| m-26 | " | " | " | " | 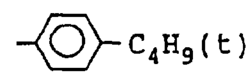 |
| m-27 | " | " | " | " | 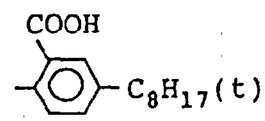 |
| m-28 | " | " | " | " | 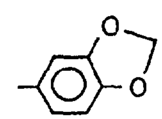 |

Methods for the preparation of couplers of the present invention are described below. The general methods of

synthesis involve the schemes indicated below.

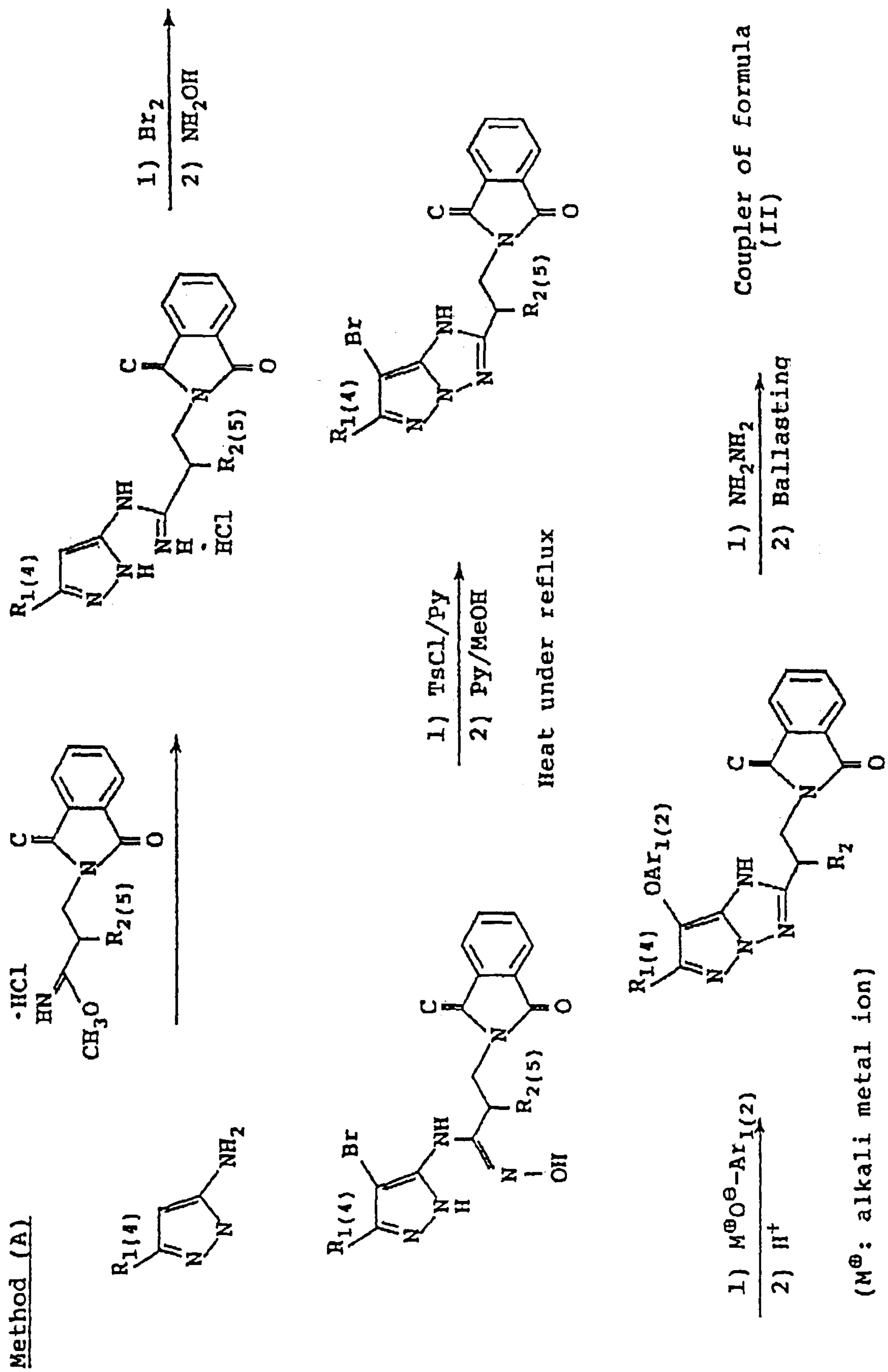

Method (B)

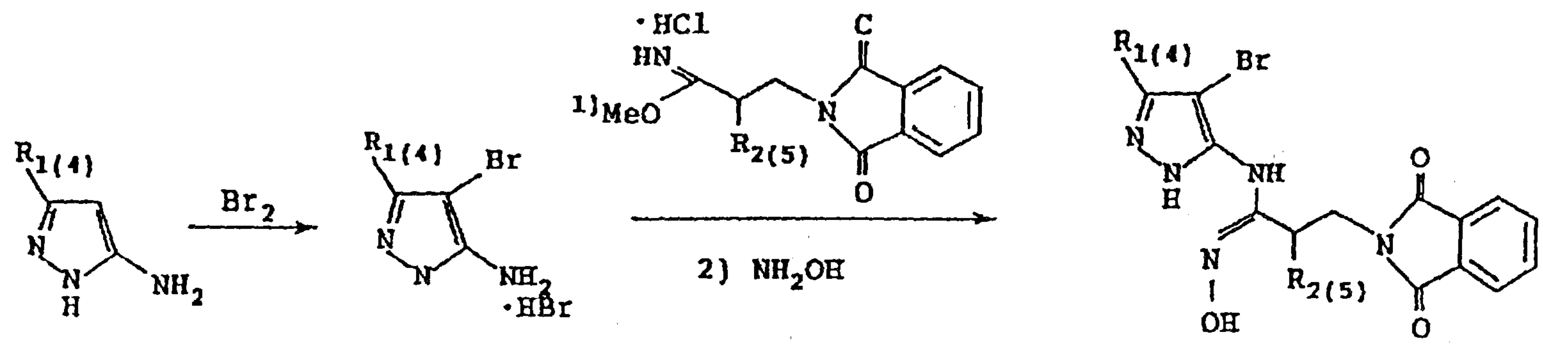


The coupler is then prepard in the same way as in method (A).

Method (C)

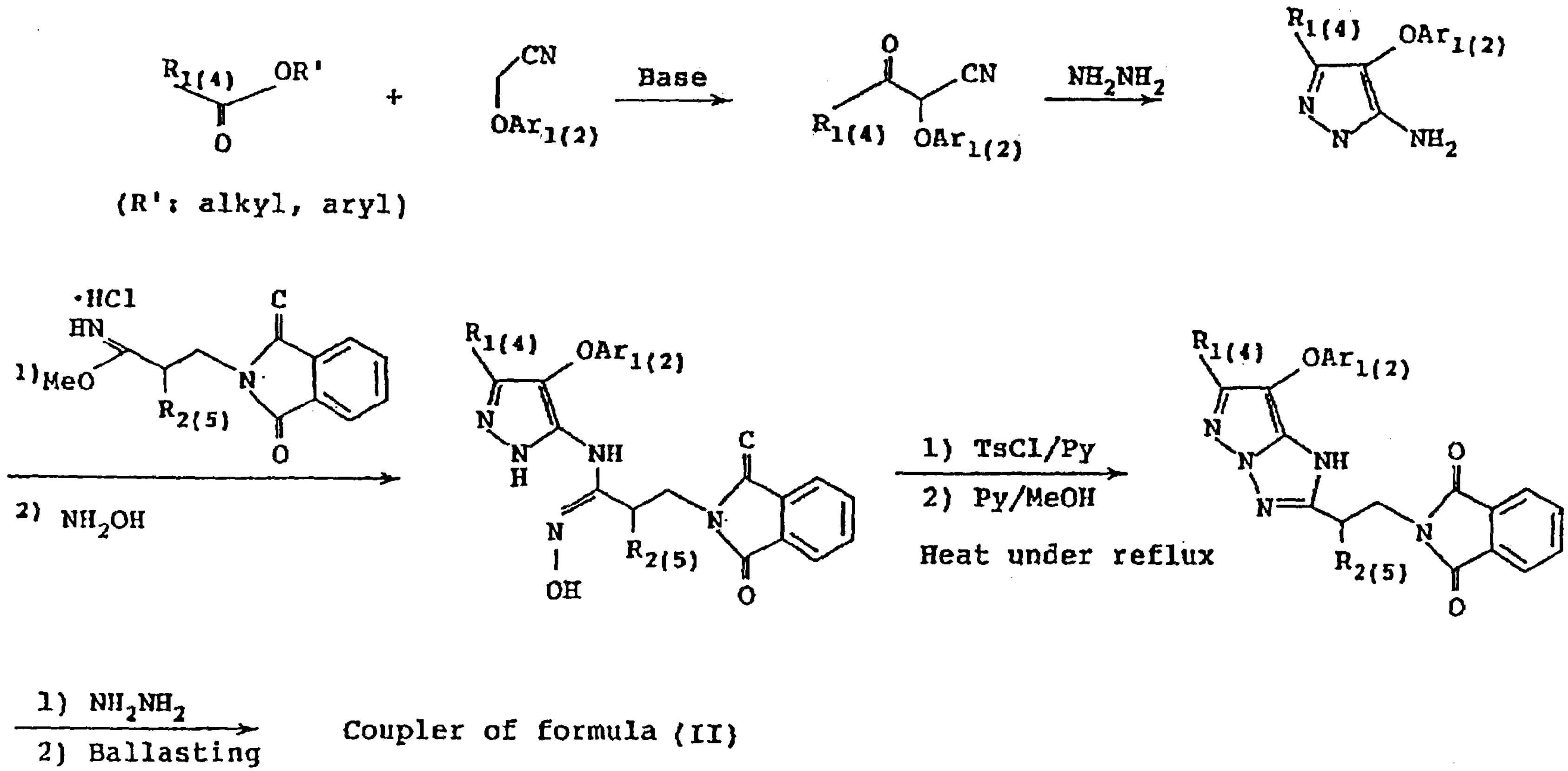
(R': alkyl, aryl)
Base
NH2NH2
1)
2) NH2OH
1) TsCl/Py
2) Py/MeOH
Heat under reflux
1) NH2NH2
2) Ballasting
Coupler of formula (II)

Example of Synthesis 2 (Preparation of Illustrative Compound m-3)

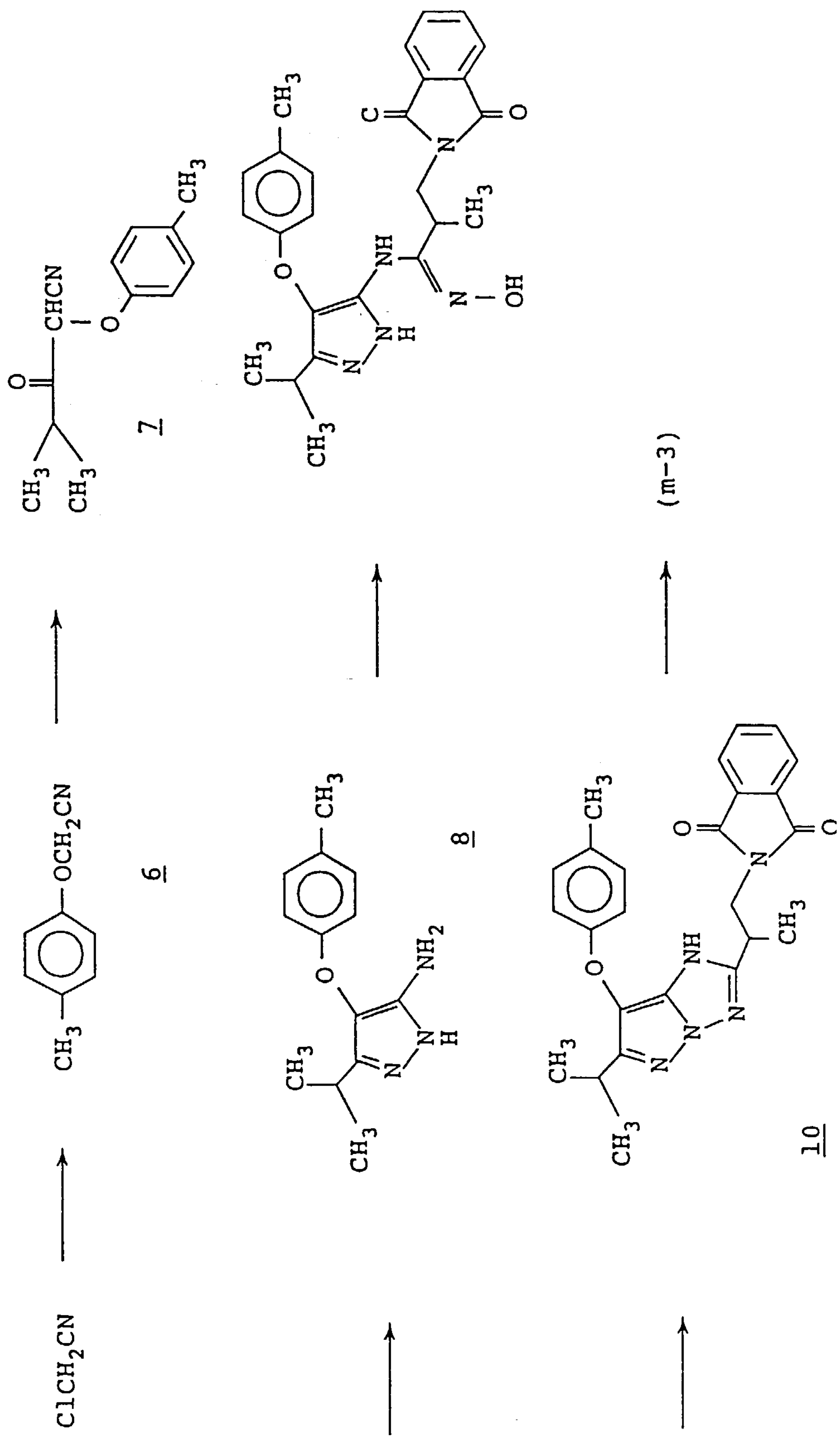

6 7 8 10 (m-3)

A 28% sodium methoxide in methanol solution (530 ml) was added dropwise (over about 10 minutes) with stirring into an ice cooled methanol (200 ml) solution of 286 grams (2.65 mol) of cresol. The ice bath -was removed and when

the mixture had warmed up to room temperature the methanol was distilled off under reduced pressure in an evaporator and adequate removal was ensured by using a high vacuum.

Chloroacetonitrile (257 grams, 2.9 mol) was added dropwise with ice cooling to a dimethylacetamide (1 -liter) solution of the sodium --salt of the cresol obtained in this way. After completing the dropwise addition, the mixture was warmed up to room temperature and stirred for about 2 hours, after which the mixture was extracted three times with an ethyl acetate/n-hexane (1:1) solvent mixture and, after washing the extract with water and saturated aqueous salt water, the extract was dried over anhydrous sodium sulfate and concentrated under reduced pressure in an evaporator. The residue was distilled under reduced pressure whereupon 335 grams of Compound 6 (110°C to 124°C/4 mm·Hg, yield 86%) was obtained.

Potassium tert-butoxide (138 grams) was added over a period of about 5 minutes with ice cooling to a solution obtained by dissolving 147 grams (1.0 mol) of Compound 6 in 1.5 liters of methyl isobutyrate. Water was added after stirring the mixture for about 1 hour and then n-hexane was added and the mixture was agitated vigorously, after which the aqueous layer was separated off and concentrated hydrochloric acid was added to adjust to a pH of about 2, after which the mixture was extracted three times with ethyl acetate and the extract was dried and concentrated, whereupon the crude product of Compound 7 was obtained as a oily substance. This crude product was separated and refined by means of silica gel column chromatography and 85 grams (yield 39%) of Compound 7 was obtained.

Next, 85 grams (0.39 mol) of Compound 7 was dissolved in 100 ml of ethanol, 19 grams (0.47 mol) of 80% hydrazine hydrate was added and the mixture was heated under reflux for about 3 hours. Subsequently, the mixture was cooled down to room temperature and crystals precipitated out after allowing the mixture to stand overnight and these crystals were recovered by filtration and dried, whereupon 76 grams (yield 84%) of Compound 8 was obtained (Melting Point 188 to 190°C).

Dimethylacetamide (500 ml) was added to 53-.5 grams (0.23 mol) of Compound 8 and the mixture was heated until a solution formed. After dissolution, the solution was cooled to about 35°C and then 86 grams (0.30 mol) of 2-methyl-phthalimidopropioimide acid methyl ester hydrochloride were added and the mixture was stirred at room temperature for about 24 hours. An aqueous solution of 32 grams (0.46 mol) of hydroxylamine hydrochloride and 19 grams (0.23 mol) of sodium acetate was then added to the reaction mixture and the mixture was heated to 50°C. The heating was stopped after about 1 hour and, after stirring for about 10 minutes as the mixture gradually returned to room temperature, the mixture was poured into 2 liters of ice with stirring and the crystals which precipitated out were recovered by filtration and washed thoroughly with water and then dried, whereupon 102 grams (yield 96%) of Compound 9 was obtained (Melting Point about 186°C (with decomposition)).

Dimethylacetamide (300 ml) was added to 101 grams (0.219 mol) of Compound 9 and the mixture was stirred. In this instance, the Compound 9 did not dissolve completely, but 43.8 grams of p-toluenesulfonyl chloride which had been dissolved in 120 ml of acetonitrile were added dropwise over a period of about 30 minutes while cooling the mixture in a water bath. After completing the dropwise addition, the mixture was stirred for about 1 hour and then 18.6 ml of pyridine was added, the water bath was removed and the mixture was stirred for about 1 hour. The reaction mixture obtained was poured into 2 liters of ice water and the aqueous solution was removed by decantation. Methanol (2 liters) was added to the residual solid material which was scratched and crushed and on filtration the tosylate of Compound 9 was obtained in the form of a colorless powder. This was immediately dispersed in 1.8 liters of methanol, and 18.6 ml of pyridine were added, then the mixture was heated to the reflux temperature and the heating was stopped when the tosylate had dissolved and the mixture was thereafter cooled gradually to room temperature with stirring. After stirring at room temperature for about 2 days, the methanol was distilled off under reduced pressure and the mixture was concentrated to about 500 ml and poured into about 2 liters of water. The powdered crystals which precipitated out were recovered by filtration and dried, whereupon 50.4 grams (yield 52%) of Compound 10 were obtained (Melting Point about 193°C (with decomposition)).

Next, 80% hydrazine hydrate (2.5 grams, 0.062 mol) was added to a solution of 15 grams (0.034 mol) of Compound 10 in 150 ml of isopropyl alcohol and, after heating the mixture under reflux for about 8 hours, the mixture was cooled to room temperature, then chloroform and saturated salt water were added and the mixture was subjected to suction filtration in order to remove the phthalhydrazide which precipitated out. The filtrate was extracted three times with chloroform, the extracts were combined, washed with saturated salt water and dried over magnesium sulfate, after which the mixture was filtrated and the amine was obtained as crystals by evaporation. This amine was dissolved in 100 ml of a 1:1 dimethylacetamide/acetonitrile mixed solvent, 4.7 ml of triethylamine were added and 14.7 grams (0.034 mol) of 2-hexyloxyethoxy-4-tert-octylbenzenesulfonyl chloride which had been dissolved in 40 ml of acetonitrile was added dropwise with stirring while cooling in ice. After the dropwise addition had been completed, the mixture was stirred for about 1 hour and then extracted and the oily material obtained after drying and evaporation was refined using silica gel column chromatography (eluent: n-hexane/ethyl acetate (4:1)) whereupon 17.4 grams (yield 72%) of the illustrative Coupler m-3 were obtained as an amorphous solid.

Examples of Syntheses 3 to 11

The melting points of couplers prepared on the basis of the general methods described in Examples of Syntheses 1 and 2 are indicated in the table below.

| Example of Synthesis No. | Illustrative Coupler | Melting Point (°C) | General Synthesis Type |
|---|---|---|---|
| 3 | M- 1 | 143 to 145 | Synthesis 1 |
| 4 | M- 7 | 189 to 192 | Synthesis 1 |
| 5 | M- 8 | 115 to 116 | Synthesis 1 |
| 6 | M- 9 | 151 to 152 | Synthesis 1 |
| 7 | M-10 | 178 to 182 | Synthesis 1 |
| 8 | M-11 | 176 to 177 | Synthesis 1 |
| 9 | M-12 | 227 to 230 | Synthesis 1 |
| 10 | M-29 | 67.5 to 70.5 | Synthesis 1 |
| 11 | m- 1 | 142 to 143 | Synthesis 2 |
| 12 | m- 2 | Amorphous (did not crystallize) | Synthesis 2 |

The couplers represented by formula (II) may be added to any photosensitive silver halide emulsion layer or non-photosensitive layer.

The amount of coupler added is from 0.01 to 2 mmol, and preferably from 0.1 mmol to 1.5 mmol, per square meter of photosensitive material.

The couplers used in the present invention can be dissolved in a high boiling point organic solvent as described hereinafter and emulsified and dispersed, or they can be emulsified and dispersed without the use of a high boiling point organic solvent.

The photosensitive materials of the present invention should have, on a support, at least one blue sensitive layer, at least one green sensitive layer and at least one red sensitive layer, but not particular limitation is imposed upon the number or order of the silver halide emulsion layers and non-photosensitive layers. Typically, silver halide photographic materials have, on a support, at least one photosensitive layer comprised of a plurality of silver halide layers which have essentially the same color sensitivity but different photographic speeds, the photosensitive layer being a unit photosensitive layer which is color sensitive to blue light, green light or red light, and in multi-layer silver halide color photographic materials the arrangement of the unit photosensitive layers generally involves the establishment of the layers in the order, from the support side, of red sensitive layer, green sensitive layer, and blue sensitive layer. However, this order may be reversed, as required, and the layers may be arranged in such a way that a layer which has a different color sensitivity is sandwiched between layers which have the same color sensitivity.

Various non-photosensitive layers, such as interlayers, may be established between the silver halide photosensitive layers, and as uppermost and lowermost layers.

The interlayers may contain couplers and DIR compounds such as those disclosed in the specifications of JP-A-61-43748, JP-A-59-113438, JP-A-59-113440, JP-A-61-20037 and JP-A-61-20038, and they may also contain conventionally anti-color mixing compounds.

The plurality of silver halide emulsion layers constituting each unit photosensitive layer is preferably a double layer structure comprised of a high speed emulsion layer and a low speed emulsion layer as disclosed in DE-C- 1,121,470 or GB-B- 923,045. Generally, arrangements in which the photographic speed is lower in the layer arranged closer to the support are preferred, and non-photosensitive layers may be established between each of the silver halide emulsion layers. Furthermore, the low speed layers may be arranged on the side farthest away from the support and the high speed layers may be arranged on the side closest to the support as disclosed, for example, in JP-A-57-112751, JP-A-62-200350, JP-A-62-206541 and JP-A-62-206543.

In practical terms, the sequence of the various layers of the photosensitive element may be, from the side located farthest from the support, low speed blue sensitive layer (BL)/high speed blue sensitive layer (BH)/high speed green sensitive layer (GH)/low speed green sensitive layer (GL)/high speed red sensitive layer (RH)/low speed red sensitive layer (RL), or alternatively, the sequence BH/BL/GL/GH/RH/RL, or the sequence BH/BL/GH/GL/RL/RH.

Furthermore, the layers can be arranged in the order, from the side located farthest from the support, in a sequence of blue sensitive layer/GH/RH/GL/RL as disclosed in JP-B-55-34932. (The term "JP-B" as used herein means an "examined Japanese patent publication".) Furthermore, the layers can also be arranged in the order, from the side located farthest away from the support, in a sequence of blue sensitive layer/GL/RL/GH/RH, as disclosed in the specifications

of JP-A-56-25738 and JB-A-62-63936.

Furthermore, arrangements are suitable, such as disclosed in JP-B-49-15495, in which there are three layers which have different speeds with the speed decreasing in the direction towards the support wherein a relatively high speed silver halide emulsion layer is arranged at the top with a silver halide emulsion layer which has a lower speed than the aforementioned top layer as an interlayer and a silver halide emulsion layer which has a relatively lower speed than the interlayer arranged as a bottom layer. In the case of structures of this type which contain three layers having different speeds, the layers in a layer of the same color sensitivity may be also arranged in the order, from the side farthest from the support, in a sequence of intermediate speed emulsion layer/high speed emulsion layer/low speed emulsion layer, as disclosed in the specification of JP-A-59-202464.

Furthermore, the layers can be arranged in the order of high speed emulsion layer/low speed emulsion layer/intermediate speed emulsion layer, or low speed emulsion layer/intermediate speed emulsion layer/high speed emulsion layer, for example.

Furthermore, the arrangement of layers may be varied in the ways suggested above in cases where there are four or more layers having different speeds.

Arrangements in which donor layers (CL) which have an interlayer effect and of which the spectral sensitivity distribution differs from that of the principal photosensitive layer such as the BL, GL, RL, for example, are adjacent to, or in the proximity of the principal photosensitive layers, as disclosed in US-A-4,663,271, 4,705,744 and 4,707,436, JB-A-62-106448 and JP-A-63-89580, are desirable for improving color reproduction.

As described above, various layer structures and arrangements can be selected respectively according to the purpose of the photosensitive material.

The preferred types of silver halides for inclusion in the photographic emulsion layers provided in the photographic photosensitive material of the present invention, which can be a color negative film or a color reversal film, are silver iodobromides, silver iodochlorides or silver iodochlorobromides which contain not more than about 30 mol% of silver iodide. Preferably, the silver halide is a silver iodobromide or silver iodochlorobromide which contains from about 2 mol% to about 25 mol% of silver iodide.

In those instances where the photographic photosensitive material of the present invention is a color printing paper, the silver halide which is included in the photographic emulsion layers is preferably a silver chlorobromide or a silver chloride which is essentially silver iodide-free. Here, the term "essentially silver iodide-free" signifies that the silver iodide content is not more than 1 mol%, and preferably not more than 0.2 mol%. -The halogen composition of these silver chlorobromide emulsions may have any silver bromide/silver chloride ratio. This ratio can extend over a wide range, depending on the application, but the use of emulsions in which the silver chloride content is at least 2 mol% is preferred. The use of so-called high silver chloride emulsions in which the silver chloride content is high is preferred for photosensitive materials which are suitable for rapid processing. The silver chloride content of these high silver chloride emulsions is preferably at least 90 mol%, and most desirably at least 95 mol%. The use of substantially pure silver chloride emulsions which have a silver chloride content of about 98 to 99.9 mol% is desirable for reducing the rate of replenishment of the development processing bath.

The silver halide grains in the photographic emulsion may have a regular crystalline form such as a cubic, octahedral or tetradecahedral form, an irregular crystalline form such as a spherical or tabular form, a form which has crystal defects such as twinned crystal planes, or a crystalline form which is a composite of these forms.

The grain size of the silver halide may be very fine, e.g., at less that about 0.2 μm, or large with a projected area diameter of up to about 10 μm, and the emulsions may be poly-disperse emulsions or mono-disperse emulsions.

The photographic emulsions which can be used in the present invention can be prepared, for example, using the methods disclosed in Research Disclosure (RD) No. 17643 (December, 1978), pages 22 to 23, "I. Emulsion Preparation and Types", and Research Disclosure No. 18716 (November 1979), page 648, by P. Glafkides in Chimie et Physique Photographique, published by Paul Montel, 1967, by G.F. Duffin in Photographic Emulsion Chemistry, published by Focal Press, 1966, and by V.L. Zelikman et al. in Making and Coating Photographic Emulsions, published by Focal Press, 1964.

The mono-disperse emulsions disclosed, for example, in US-A- 3,574,628 and 3,655,394, and GB-B- 1,413,748 are also highly suitable.

Furthermore, tabular grains which have an aspect ratio of at least about 5 can be used in the present invention. Tabular grains can be prepared easily using the methods described, for example, in Gutoff, Photographic Science and Engineering, Volume 14, pages 248 to 257 (1970), US-A- 4,434,226, 4,414,310, 4,433,048 and 4,439,520, and GB-B- 2,112,157.

The crystal structure may be uniform, or the interior and exterior parts of the grains may have different halogen compositions, or the grains may have a stratified or layered structure. Moreover, silver halides which have different compositions may be joined with an epitaxial junction or they may be joined with compounds other than silver halides, such as silver thiocyanate or lead oxide, for example. Furthermore, mixtures of grains which have various crystalline forms can be used.

The silver halide emulsions which can be used generally are subjected to physical ripening, chemical ripening and spectral sensitization. Additives which can be used in such processed have been disclosed in Research Disclosure, Nos. 17643 and 18716, and the locations of relevant disclosures therein are summarized in the reference table provided below.

The use of non-photosensitive fine grained silver halides is desirable in the- practice of the present invention. Non-photosensitive fine grained silver halides are fine grained silver halides which are not photosensitive at the time of the imagewise exposure for obtaining the dye image and which undergo essentially no development during development processing. Non-photosensitive fine grained silver halides which have not been pre-fogged are preferred.

The non-photosensitive fine grained silver halide has a silver bromide content from 0 to 100 mol%, containing silver chloride and/or silver iodide as required. Those which have a silver iodide content of from 0.5 to 10 mol% are preferred.

The fine grained silver halide preferably has an average grain size (the average value of the diameters of the circles corresponding to the projected areas) of from 0.01 to 0.5 μm, and most desirably the average grain size is from 0.02 to 0.2 μm.

The fine grained silver halide can be prepared using the same methods as used in general for the preparation of photosensitive silver halides. In this case, the surface of the silver halide grains does not need to be optically sensitized and neither is there any need for spectral sensitization. However, the pre-addition of known stabilizers such as triazole, azaindene, benzothiazolium or mercapto based compounds before addition to the film coating liquid is desirable.

Known photographically useful additives which can be used in the present invention are also disclosed in the two Research Disclosure, Nos. 17643 and 18716 referred to above, and the locations of the relevant disclosures therein are also indicated in the reference table provided below.

| | Type of Additive | RD 17643 | RD 18716 |
|---|---|---|---|
| 1. | Chemical sensitizers | Page 23 | Page 648, right column |
| 2. | Speed increasing agents | | As above |
| 3. | Spectral sensitizers and super-sensitizers | Pages 23 to 24 | Page 648, right column to page 649, right column |
| 4. | Whiteners | Page 24 | |
| 5. | Anti-foggants and stabilizers | Pages 25 to 26 | Page 649, right column |
| 6. | Light absorbers, filter dyes and ultra-violet absorbers | Pages 25 to 26 | Page 649, right column to page 650, left column |
| 7. | Anti-staining agents | Page 25, right column | Page 650, left column to right column |
| 8. | Dye image stabilizers | Page 25 | |
| 9. | Film hardening agents | Page 26 | Page 651, left column |
| 10. | Binders | Page 26 | As above |
| 11. | Plasticizers, lubricants | Page 27 | Page 650, right column |
| 12. | Coating promotors, surfactants | Pages 26 to 27 | Page 650, right column |
| 13. | Anti-static agents | Page 27 | As above |

Furthermore, the addition to the photosensitive material of the compounds which can react with and fix formaldehyde such as disclosed in US-A- 4,411,987 and 4,435,503 is desirable for preventing the deterioration of photographic performance due to formaldehyde gas.

Different types of color couplers can be used in the present invention, and specific examples thereof are disclosed in the patents cited in the aforementioned Research Disclosure, (RD) No. 17643, sections VII-C-G.

Those disclosed, for example, in US-A-3,933,501, 4,022,620, 4,326,024, 4,401,752 and 4,248,961, JP-B-58-10739, GB-B 1,425,020 and 1,467,760, US-A- 3,973,968, 4,314,023 and 4,511,649, and EP-A- 249,473 are preferred as yellow couplers.

5-Pyrazolone based compounds and pyrazoloazole based compounds are preferred as magenta couplers other than the magenta couplers of the present invention, and those disclosed, for example, in US-A- 4,310,619 and 4,351,897, EP-B- 73,636, US-A- 3,061,432 and 3,725,067, Research Disclosure, No. 24220 (June 1984), JP-A-60-33552, Research Disclosure, No. 24230 (June 1984), JP-A-60-43659, JP-A-61-72238, JP-A- 60-35730, JP-A-55-118034, JP-A-60-185951, US-A- 4,500,630, 4,540,654 and 4,556,630, and International Patent WO 88/04795 are especially desirable.

Phenol and naphthol based couplers can be used as cyan couplers, and preferred examples thereof are those disclosed, for example, in US-A- 4,052,212, 4,146,396, 4,228,233, 4,296,200, 2,369,929, 2,801,171, 2,772,162, 2,895,826, 3,772,002, 3,758,308, 4,334,011 and 4,327,173, DE-A- 3,329,729, EP-A- 121,365 and 249,453, US-A-3,446,622, 4,333,999, 4,775,616, 4,451,559, 4,427,767, 4,690,889, 4,254,212 and 4,296,199, and JP-A-61-42658.

The colored couplers for correcting the unwanted absorptions of colored dyes are preferably those disclosed, for example, in section VII-G of Research Disclosure, No. 17643, US-A- 4,163,670, JP-B-57-39413, US-A- 4,004,929 and 4,138,258, and GB-B- 1,146,368 are preferred. Furthermore, it is also desirable to use couplers which correct the unwanted absorption of colored dyes by means of fluorescent dyes which are released upon coupling such as disclosed in US-A- 4,774,181, and couplers which have, as split-off groups, dye precursor groups which can form dyes upon reaction with the developing agent such as are disclosed in US-A- 4,777,120.

The couplers disclosed in US-A- 4,366,237, GB-B 2,125,570, EP-B- 96,570 and DE-A- 3,234,533 are preferred as couplers of which the colored dyes have a suitable degree of diffusibility.

Typical examples of polymerized dye forming couplers have been disclosed, for example, in US-A- 3,451,820, 4,080,211, 4,367,282, 4,409,320 and 4,576,910, and GB-B- 2,102,173.

The use of couplers which release photographically useful residual groups upon coupling is desirable in the practice of the present invention. The DIR couplers which release development inhibitors such as disclosed in the patents cited in section VII-F of the aforementioned Research Disclosure, 17643, JP-A-57-151944, JP-A-57-154234, JP-A-60-184248, JP-A-63-37346, JP-A-63-37350 and US-A- 4,248,962 and 4,782,012 are preferred.

The couplers disclosed in British Patents 2,097,140 and 2,131,188, JP-A-59-157638 and JP-A-59-170840 are preferred as couplers which release nucleating agents or development accelerators in the form of the image during development.

Other compounds which can be used in photosensitive materials of the present invention include the competitive couplers disclosed, for example, in US-A- 4,130,427, the poly-equivalent couplers disclosed, for example, in US-A-4,283,472, 4,338,393 and 4,310,618, the DIR redox compound releasing couplers, DIR coupler releasing couplers, DIR coupler releasing redox compounds or DIR redox releasing redox compounds disclosed, for example, in JP-A-60-185950 and JP-A-62-24252, the couplers which release dyes in which the color is restored after split-off such as disclosed in EP-A- 173,302, the bleach accelerator releasing couplers disclosed, for example, in Research Disclosure, No. 11449, ibid., No. 24241, and JP-A-61-201247, the ligand releasing couplers disclosed, for example, in US-A-4,553,477, the leuco dye releasing couplers such as disclosed in JP-A-63-75747, and the couplers which release fluorescent dyes such as disclosed in US-A- 4,774,181.

The couplers which are used in the present invention can be introduced into the photosensitive material using a variety of known methods of dispersion.

Examples of high boiling point solvents which can be used in the oil-in-water dispersion method have been disclosed, for example, in US-A- 2,322,027.

Specific examples of high boiling point organic solvents which have a boiling point of at least 175°C at atmospheric pressure which can be used in the oil-in-water dispersion method include phthalic acid esters (for example, dibutyl phthalate, dicyclohexyl phthalate, di-2-ethylhexyl phthalate, decyl phthalate, bis(2,4-di-tert-amylphenyl)phthalate, bis (2,4-di-tert-amylphenyl)isophthalate and bis(1,1-diethylpropyl)phthalate), phosphate or phosphonate esters (for example, triphenyl phosphate, tricresyl phosphate, 2-ethylhexyldiphenyl phosphate, tricyclohexyl phosphate, tri-2-ethylhexyl phosphate, tridodecyl phosphate, tributoxyethyl phosphate, trichloropropyl phosphate and di-2-ethylhexylphenyl phosphonate), benzoic acid esters (for example, 2-ethylhexyl benzoate, dodecyl benzoate, 2-ethylhexyl p-hydroxybenzoate), amides (for example, N,N-diethyldodecanamide, N,N-diethyllaurylamide and N-tetradecylpyrrolidone), alcohols or phenols (for example, isostearyl alcohol and 2,4-di-tert-amylphenol), aliphatic carboxylic acid esters (for example, bis(2-ethylhexyl)sebacate, dioctyl azelate, glycerol tributyrate, isostearyl lactate and trioctyl citrate), aniline derivatives (for example, N,N-dibutyl-2-butoxy-5-tert-octylaniline) and hydrocarbons (for example, paraffins, dodecylbenzene and diisopropylnaphthalene). Furthermore, organic solvents which have a boiling point above about 30°C, and preferably of at least 50°C, but below about 160°C can be used as auxiliary solvents, and typical examples of these solvents include ethyl acetate, butyl acetate, ethyl propionate, methyl ethyl ketone, cyclohexanone, 2-ethoxyethyl acetate and dimethylformamide.

Actual examples of the processes and effects of the latex dispersion method and of latexes for loading purposes have been disclosed, for example, in US-A- 4,199,363, and DE-A- 2,541,274 and 2,541,230.

Furthermore, these couplers can be loaded onto a loadable latex in the presence or absence of the aforementioned high boiling point organic solvents (for example, US-A- 4,203,716), or they can be dissolved in a water insoluble but organic solvent soluble polymer and emulsified and dispersed in an aqueous hydrophilic colloid solution.

Furthermore, use of the homopolymers or copolymers disclosed on pages 12 to 30 of the specification of International Patent WO88/00723 is preferred. The use of acrylamide based polymers is especially desirable from the viewpoint of dye stabilization.

The addition to the color photosensitive materials of the present invention of various fungicides and bactericides such as 1,2-benzisothiazolin-3-one, n-butyl p-hydroxybenzoate, phenol, 4-chloro-3,5-dimethylphenol, 2-phenoxyethanol and 2-(4-thiazolyl)benzimidazole as disclosed in JP-A-63-257747, JP-A-62-272248 and JP-A-1-80941 is desirable.

The present invention can be applied to a variety of color photosensitive materials. Typical examples include color negative films for cinematographic purposes, color reversal films for slides and television purposes, color papers, color positive films and color reversal papers.

Suitable supports which can be used in the present invention have been disclosed, for example, on page 28 of the aforementioned Research Disclosure, No. 17643, and from the right hand column of page 647 to the left hand column of page 648 of Research Disclosure, No. 18716.

The photosensitive materials of the present invention are selected such that the total film thickness of all the hydrophilic colloid layers arranged on the same side as the emulsion layers is preferably not more than 28 μm, more desirably not more than 23 μm, and most desirably not more than 18 μm. Furthermore, the film swelling rate $T_{1/2}$ is preferably not more than 30 seconds and most desirably not more than 20 seconds. Here, the film thickness signifies the film thickness measured under conditions of 25°C, 55% relative humidity (2 days) and the film swelling rate $T_{1/2}$ is that measured using the methods well known to those in the industry. For example, measurements can be made using a swellometer of the type described by A. Green in Photogr. Sci. Eng., Volume 19, Number 2, pages 124 to 129, and $T_{1/2}$ is defined as the time taken to reach half the saturated film thickness, taking 90% of the maximum swelled film thickness reached on processing the material for 3 minutes 15 seconds in a color development bath at 30°C as the saturated film thickness.

The film swelling rate $T_{1/2}$ can be adjusted by adding film hardening agents for the gelatin which is used as a binder, or by changing the ageing conditions after coating. Furthermore, the swelling factor is preferably from 150% to 400%. The swelling factor can be calculated from the maximum swelled film thickness obtained under the conditions described above using the expression (maximum swelled film thickness minus film thickness)/film thickness.

Color photographic photosensitive materials which are in accordance with the present invention can be developed and processed using the conventional methods disclosed on pages 28 to 29 of the aforementioned Research Disclosure, No. 17643 and from the left hand column to the right hand column of page 615 of the aforementioned Research Disclosure, No. 18716.

The color development baths used in the development processing of photosensitive materials of the present invention are preferably aqueous alkaline solutions which contain a primary aromatic amine based color developing agent as the principal component. Aminophenol based compounds are also useful as color developing agent, but the use of p-phenylenedimine based compounds is preferred, and typical examples include 3-methyl-4-amino-N,N-diethylaniline, 3-methyl-4-amino-N-ethyl-N-β-hydroxyethylaniline, 3-methyl-4-amino-N-ethyl-N-β-methanesulfonamidoethylaniline, 3-methyl-4-amino-N-ethyl-N-β-methoxyethylaniline, and the sulfate, hydrochloride and p-toluenesulfonate salts of these compounds. From among these compounds, 3-methyl-4-amino-N-ethyl-N-β-hydroxyethylaniline sulfate is especially preferred. Two or more of these compounds can be used conjointly, according to the intended purpose.

The color development bath generally contains pH buffers such as alkali metal carbonates, borates or phosphates, and development inhibitors or anti-foggants such as chlorides, bromides, iodides, benzimidazoles, benzothiazoles or mercapto compounds. The development bath may also contain, as required, various preservatives such as hydroxylamine, diethylhydroxylamine, sulfite, hydrazines such as N,N-biscarboxymethylhydrazine, phenylsemicarbazides, triethanolamine and catecholsulfonic acids, organic solvents such as ethylene glycol and diethylene glycol, development accelerators such as benzyl alcohol, polyethylene glycol, quaternary salts and amines, dye forming quaternary ammonium salts and amines, dye forming couplers, competitive. couplers, auxiliary developing agents such as 1-phenyl-3-pyrazolidone, thickeners and various chelating agents as typified by the aminopolycarboxylic acids, aminopolyphosphonic acids, alkylphosphonic acids and phosphonocarboxylic acids, typical examples of which include ethylenediaminetetraacetic acid, nitrilotriacetic acid, diethylenetriaminepentaacetic acid, cyclohexanediaminetetraacetic acid, hydroxyethyliminodiacetic acid, 1-hydroxyethylidene-1,1-diphosphonic acid, nitrilo-N,N,N-trimethylenephosphonic acid, ethylenediamine-N,N,N,N-tetramethylenephosphonic acid, ethylenediaminedi(o-hydroxyphenylacetic acid) and salts of these acids.

Furthermore, color development is carried out after a normal black-and-white development in the case of reversal processing. Known black-and-white developing agents including dihydroxybenzenes such as hydroquinone, 3-pyrazolidones such as 1-phenyl-3-pyrazolidone, and aminophenols such as N-methyl-p-aminophenol, for example, can be used individually, or in combinations thereof, in the black-and-white developer.

The pH of these color development baths and black-and-white developing baths is generally from 9 to 12. Furthermore, the replenishments rate for these developers depends on the color photographic photosensitive material which is being processed but, in general, is not more than 3 liters per square meter of photosensitive material and the replenishment rate can be set to not more than 500 ml by reducing the bromide ion concentration in the replenisher. In cases where the replenishment rate is low, it is desirable that evaporation and aerial oxidation of the liquid should be prevented by minimizing the area of contact with the air in the processing tank.

The contact area between the air and the photographic processing bath in a processing tank can be represented by the open factor which is defined below.

$$\text{Open Factor} = \frac{\text{Processing bath and Air Contact Area (cm}^2\text{)}}{\text{Processing Bath Volume (cm}^3\text{)}}$$

The above mentioned open factor is preferably less than 0.1, and most preferably from 0.001 to 0.05. As well as the establishment of a shielding material such as a floating lid, for example, on the surface of the photographic processing bath in the processing tank, the method involving the use of a movable lid -as disclosed in JP-A-1-82033 and the method involving the slit development processing disclosed in JP-A-63-216050 can be used as means of reducing the open factor. Reduction of the open factor is preferably applied not only to the processes of color development and black-and-white development but also to all the subsequent processes, such as the bleaching, bleach-fixing, fixing, water washing and stabilization processes. Furthermore, the replenishment rate can be reduced by using some means of suppressing the accumulation of bromide ion in the development bath.

The color development processing time is generally established between 2 and 5 minutes, but shorter processing times can be devised by increasing the pH or by increasing the concentration of the color developing agent.

The photographic emulsion layer is subjected to a conventional bleaching process after color development. The bleaching process may be carried out at the same time as a fixing process (in a bleach-fix process) or it may be carried out as a separate process from the fixing process. Moreover, a bleach-fix process also can be carried out after a bleaching process in order to speed up processing. Moreover, processing can be carried out in two connected bleach-fix baths, a fixing process can be carried out before a bleach-fixing process or a bleaching process can be carried out after a bleach-fix process, as required. Compounds of multivalent metals, such as iron(III), for example, peracids, quinones and nitro compounds can be used as bleaching agents. Typical bleaching agents include organic complex salts of iron(III), for example, complex salts with aminopolycarboxylic acids such as ethylenediaminetetraacetic acid, diethylenetriaminepentaacetic acid, cyclohexanediaminetetraacetic acid, methyliminodiacetic acid, 1,3-diaminopropanetetraacetic acid and glycol ether diaminetetraacetic acid, or citric acid, tartaric acid or malic acid. From among these materials, the use of polyaminocarboxylic acid iron(III) complex salts, principally ethylenediaminetetraacetic acid iron(III) complex salts and 1,3-diaminopropanetetraacetic acid iron(III) salts, is preferred from the points of view of both rapid processing and the prevention of environmental pollution. Moreover, the aminopolycarboxylic acid iron(III) complex salts are especially useful in both bleach baths and bleach-fix baths. The pH value of the bleach baths and bleach-fix baths in which these aminopolycarboxylic acid iron(III) salts are used is generally from 4.0 to 8, but lower pH values can be used in order to speed up processing.

Bleaching accelerators can be used, as required, in the bleach baths, bleach-fix baths or bleach or bleach-fix prebaths. Specific examples of useful bleach accelerators are disclosed in the following specifications: compounds which have a mercapto group or a disulfide group disclosed, for example, in US-A- 3,893,858, DE-C- 1,290,812 and 2,059,988, JP-A-53-32736, JP-A-53-57831, JP-A-53-37418, JP-A-53-72623, JP-A-53-95630, JP-A-53-95631, JP-A-53-104232, JP-A-53-124424, JP-A-53-141623, JP-A-53-28426, and Research Disclosure, No. 17129 (June 1978); the thiazolidine derivatives disclosed in JP-A-50-140129; the thiourea derivatives disclosed in-JP-B-45-8506, JP-A-52-20832, JP-A-53-32735 and US-A- 3,706,561; the iodides disclosed in DE-C- 1,127,715 and JP-A-58-16235; the polyoxyethylene compounds disclosed in DE-C- 966,410 and 2,748,430; the polyamine compounds disclosed in JP-B-45-8836; other accelerator compounds variously disclosed in JP-A-49-42434, JP-A-49-59644, JP-A-53-94927, JP-A-54-35727, JP-A-55-26506 and JP-A-58-163940; and the bromide ion. From among these compounds those which have a mercapto group or a disulfide group are preferred in view of their large accelerating effect, and the compounds disclosed in US-A- 3,893,858, West German Patent 1,290,812 and JP-A-53-95630 are especially preferred. Moreover, the compounds disclosed in US-A- 4,552,834 are also desirable. These bleaching accelerators may also be added to the photosensitive materials. These bleaching accelerators are especially effective when bleach-fixing camera color photosensitive materials.

The inclusion of organic acids as well as the compound indicated above in the bleach baths and bleach-fix baths is desirable for preventing the occurrence of bleach staining. Compounds which have an acid dissociation constant (pka) of from 2 to 5 are especially desirable as organic acids, and actual examples include acetic acid and proionic acid.

Thiosulfates, thiocyanates, thioether based compounds, thioureas and large amounts of iodide can be used, for example, as the fixing agent which is used in a fixing bath or bleach-fix bath. Thiosulfates are generally used and ammonium thiosulfate in particular can be used in the widest range of -applications. Furthermore, the combined use of thiosulfate and thiocyanate, thioether compounds, thiourea or iodide is also desirable. Sulfites, bisulfites, carbonyl/bisulfite addition compounds or the sulfinic acid compounds disclosed in EP-A- 294,769 are preferred as preservatives for fixing baths and bleach-fix baths. Moreover, the addition of various aminopolycarboxylic acids and organophosphonic acids to the fixing baths and bleach-fixing baths is desirable for stabilizing these baths. The total time of the de-silvering process is preferably as short as possible within the range where de-silvering failure does not occur. The preferred de-silvering time is from 1 to 3 minutes, and most desirably the de-silvering time is from 1 to 2 minutes. Furthermore, the processing temperature usually is from 25°C to 50°C, and preferably from 35°C to 45°C. The de-silvering rate is improved and the occurrence of staining after processing is effectively prevented when processing is

conducted within the above preferred temperature range.

The de-silvering baths are preferably agitated as strongly as possible during the de-silvering processing. Specific examples of methods of strong agitation include the methods in which a jet of processing bath is directed onto the emulsion surface of the photosensitive material as disclosed in JP-A-62-183460 and JP-A-62-183461; the methods in which the agitation effect is increased using a rotary device as disclosed in JP-A-62-183461; the methods in which the photosensitive material is moved with a wiper blade which is established in the bath in contact with the emulsion surface and the agitation effect is increased by the generation of turbulence at the emulsion surface; and the methods in which the circulating flow rate of the processing bath as a whole is increased. These means of increasing agitation are effective in bleach baths, bleach-fix baths and fixing baths. It is thought that increased agitation increases the rate of supply of bleaching agent and fixing agent to the emulsion film and consequently increases the de-silvering rate. Furthermore, the aforementioned means of increasing agitation are more effective in cases where a bleaching accelerator is being used, and they may provide a marked increase in the accelerating effect and eliminate the fixer inhibiting action of the bleaching accelerator.

The automatic processors used for photosensitive materials of the present invention preferably have photosensitive material transporting devices as disclosed in JP-A-60-191257, JP-A-60-191258 or JP-A-60-191259. With such a transporting device, -such as that disclosed in the aforementioned JP-A-60-191257, the carry over of processing bath solution from one bath to the next bath is greatly reduced and this is very effective for preventing deterioration in processing bath performance. These effects are especially useful for shortening the processing time in each process and for reducing the replenishment rate needed for each processing bath.

The silver halide color photographic materials of this invention are generally subjected to a water washing process and/or stabilization process after the de-silvering process. The amount of wash water used in the washing process can be fixed within a wide range, depending on the application and the nature of the photosensitive material (for example, depending on the materials such as couplers which have been used), and processing conditions such as the wash water temperature, the number of water washing tanks (the number of water washing stages) and the replenishment system, i.e., whether a counter flow or a sequential flow system is used, and various other processing conditions. The relationship between the amount of water used and the number of washing tanks in a multi-stage counter-flow system can be obtained using the method outlines on pages 248 to 253 of the Journal of the Society of Motion Picture and Television Engineers, Volume 64 (May 1955).

The amount of wash water used can be greatly reduced by using the multi-stage counter-flow system noted in the aforementioned literature, but with the concomitant drawbacks that bacteria proliferate due to the increased residence time of the water in the tanks and problems arise with respect to suspended matter which is produced in the tanks which undesirably attaches to the photosensitive material. The method in which the calcium ion and magnesium ion concentrations are reduced, as disclosed in JP-A-62-288838, is very effective as a means of overcoming this problem when processing color photosensitive materials of the present invention. Furthermore, the isothiazolone compounds and thiabendazoles disclosed in JP-A-57-8542, the chlorine based disinfectants such as chlorinated sodium isocyanurate, and benzotriazole, for example, and the disinfectants disclosed in The Chemistry of Biocides and Fungicides by Horiguchi, (1986, Sanko Shuppan), in Killing Micro-organisms, Biocidal and Fungicidal Techniques (1982) published by the Health and Hygiene Technology Society, and in A Dictionary of Biocides and Fungicides (1986) published by the Japanese Biocide and Fungicide Society, can also be used in this regard.

The pH value of the washing water when processing photosensitive materials of the present invention is from 4 to 9, and preferably from 5 to 8. The washing water temperature and the washing time can be set variously in accordance with the nature and application of the photosensitive material but, in general, washing conditions of from 20 seconds to 10 minutes at a temperature of from 15°C to 45°C, and preferably of from 30 seconds to 5 minutes at a temperature of from 25°C to 40°C, are selected. Moreover, the photosensitive materials of this invention can be processed directly in a stabilizing bath instead of being subjected to a water wash as described above. The known methods disclosed in JP-A-57-8543, JP-A-58-14834 and JP-A-60-220345 can be used for a stabilization process of this type.

Furthermore, in some cases, a stabilization process is carried out following the aforementioned water washing process, and the stabilizing baths which contain a dye stabilizing agent and surfactant which are used as final baths with camera color photosensitive materials are an example of such a process. Aldehydes such as formalin and glutaraldehyde, N-methylol compounds, hexamethylenetetramine and aldehyde/ bisulfite addition compounds can be used, for example, as dye stabilizing agents.

Various chelating agents and fungicides can also be added to these stabilizing baths.

The overflow which accompanies replenishment of the above mentioned water washing or stabilizing baths can be reused in other processes, such as the de-silvering process, for example.

Concentration correction with the addition of water is desirable in cases where the above mentioned processing baths become concentrated by evaporation when processing in an automatic processor, for example.

Color developing agents can be incorporated into a silver halide color photosensitive material of the present invention with a view to simplifying and speeding up processing. The incorporation of various color developing agent

precursors is preferred. For example, the indoaniline based compounds disclosed in US-A- 3,342,597, the Shiff's base type compounds disclosed in US-A- 3,342,599, Research Disclosure, No. 14850 and Research Disclosure, No. 15159, the aldol compounds disclosed in Research Disclosure, No. 13924, the metal complex salts disclosed in US-A-3,719,492 and the urethane based compounds disclosed-in JP-A-53-135628 can be used for this purpose.

Various 1-phenyl-3-pyrazolidones may be incorporated, as required, into a silver halide color photosensitive material of the present invention with a view to accelerating color development. Typical compounds have been disclosed, for example, in JP-A-56-64339, JP-A-57-144547 and JP-A-58-115438.

The various processing baths in the present invention are used at a temperature of from 10°C to 50°C. The standard temperature is generally from 33°C to 38°C, but accelerated processing and shorter processing times can be realized at higher temperatures while, on the other hand, increased picture quality and better processing bath stability can be achieved at lower temperatures. Furthermore, processes using hydrogen peroxide intensification or cobalt intensification as disclosed in DE-C 2,226,770 or US-A- 3,674,499 can be used in order to economize on silver in the photosensitive material.

Furthermore, the silver halide photosensitive materials of the present invention can be used as the heat developable photosensitive materials disclosed, for example, in US-A- 4,500,626, JP-A-60-133449, JP-A-59-218443, JP-A-61-238056 and EP- 210,660A2.

The invention is described in more detail below by means of Examples, but the invention is not to be construed as limited by the Examples.

EXAMPLE 1

The coupler m-2 or m-4 of the present invention was weighed out in amount of 0.02 mol and 16.1 grams of a high boiling point organic solvent, namely, dibutyl phthalate, and 24 ml of ethyl acetate were added to form a solution which was thereafter emulsified and dispersed in 200 grams of a 10 wt% aqueous gelatin solution which contained 1.5 grams of sodium dodecylbenzenesulfonate (the average particle diameter of the emulsified dispersion at this stage was 0.12μ)

Then, the emulsified dispersion in its entirety was added to 247 grams of a high silver chloride emulsion (silver 70.0 gram/kg·emulsion, silver bromide content 0.5 mol%), and coated on a triacetate film base on which an underlayer had been established in such a way that the coated silver weight was 1.73 g/$m^2$. A gelatin layer was then established so as to provide a dry film thickness of 1.0 μ over the emulsion layer as a protective layer to thereby obtain Samples 102 and 103. Moreover, 1-oxy-3,5-dichloro-s-triazine sodium salt was used as a gelatin film hardening agent.

Furthermore, Comparative Samples (I) and (II) were prepared using equimolar amounts of the Comparative Couplers (A) and (B) described below respectively. The tests described below were carried out using the above samples.

Comparative Coupler (A)

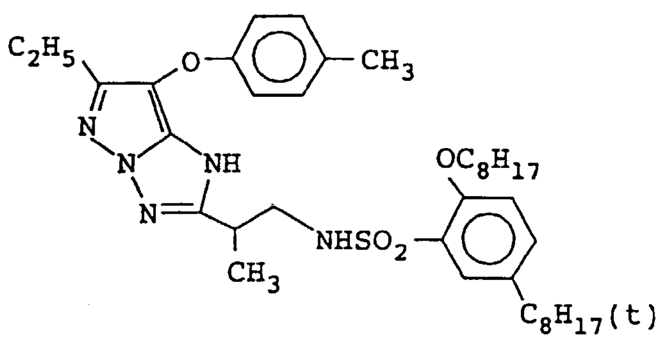

(Comparative Coupler (A) is a compound which is disclosed in JP-A-62-89961)

Comparative Coupler (B)

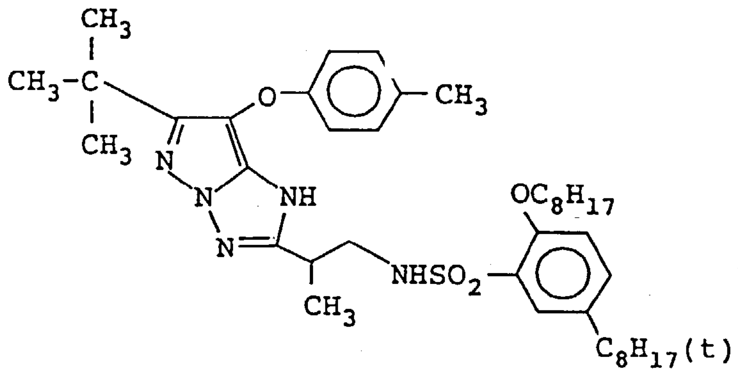

(Comparative Coupler (B) is a compound which is disclosed in European Patent 326,406A)

First, each sample was subjected to a graded exposure with a tri-color separation filter for sensitometric purposes using a sensitometer (model FWH, made by Fuji Photo Film Co., Ltd., light source color temperature 3200K). The exposure at this time was 250 CMS with an exposure time of 0.1 second.

The exposed samples were processed in an automatic processor using the processing operations and processing bath compositions indicated below.

| Processing Operation | Temperature (°C) | Time |
|---|---|---|
| Color Development | 37 | 3 min. 30 sec. |
| Bleach-fix | 33 | 1 min. 30 sec. |
| Water Wash | 24 to 34 | 3 min. |
| Drying | 70 to 80 | 1 min. |

The composition of each processing bath was as indicated below.

Color Development Bath

| | |
|---|---|
| Water | 800 ml |
| Diethylenetriaminepentaacetic acid | 1.0 g |
| Nitrilotriacetic acid | 2.0 g |
| Benzyl alcohol | 15 ml |
| Diethylene glycol | 10 ml |
| Sodium sulfite | 2.0 g |
| Potassium bromide | 1.0 g |
| Potassium carbonate | 30 g |
| N-Ethyl-N-(β-methanesulfonamidoethyl)-3-methyl-4-aminoaniline sulfate | 4.5 g |
| Hydroxylamine sulfate | 3.0 g |
| Fluorescent whitener (WHITEX 4B, made by Sumitomo Chemical Company, Limited) | 1.0 g |
| Water | to make up to 1000 ml |
| pH (25°C) | 10.25 |

Bleach-Fix Bath

| | |
|---|---|
| Water | 400 ml |
| Ammonium thiosulfate (70%) | 150 ml |
| Sodium sulfite | 18 g |
| Ethylenediaminetetraacetic acid ferric ammonium salt | 55 g |
| Ethylenediaminetetraacetic acid disodium salt | 5 g |
| Water | to make up to 1000 ml |
| pH (25°C) | 6.70 |

The results obtained on measuring the transmission spectrum of each sample were as shown in Table 1.

TABLE 1

| Sample | Coupler | Transmission Spectrum $\lambda_{max}$ | |
|---|---|---|---|
| 101 | M-4 | 543.5 nm | Present Invention |
| 102 | m-2 | 544.3 nm | Present Invention |
| 103 | m-4 | 545.0 | Present Invention |
| Comp. I | Comp. A | 548.3 | Comp. Example |
| Comp. II | Comp. B | 549.9 | Comp. Example |

As shown in Table 1, the magenta dyes obtained on processing the samples containing a coupler of the present invention had transmission spectrums which were in the preferred wavelength range of from 540 to 545 nm and this is clearly desirable from the viewpoint of obtaining optimum color reproduction. On the other hand, the couplers used in the Comparative Samples had transmission spectra outside the preferred range.

EXAMPLE 2

A multi-layer color printing paper of which the layer structure is described below was prepared on a paper support which had been laminated on both sides with polyethylene. The coating liquids were prepared in the manner described below.

Preparation of the First Layer Coating Liquid

Ethyl acetate (27.2 cc) and 8.2 grams of solvent (Solv-1) were added to 19.1 grams of yellow coupler (ExY) and 4.4 grams of color image stabilizer (Cpd-l) and 0.7 gram of color image stabilizer (Cpd-7) to form a solution which was then emulsified and dispersed in 185 cc of a 10% aqueous gelatin solution which contained 8 cc of 10% sodium dodecylbenzenesulfonate. On the other hand, the blue sensitive sensitizing dyes indicated below were added to a silver chlorobromide emulsion mixture (a 3:7 (Ag mol ratio) of two cubic emulsions of average grain sizes 0.88 μm and 0.70 μm; the variation coefficients of the grain size distributions were 0.08 and 0.10, and each emulsion had 0.2 mol% silver bromide included locally on the surface of the grains) in amounts of $2.0\times10^{-4}$ mol of each dye per mol of silver for the emulsion which had large grains and in amounts of $2.5\times10^{-4}$ mol of each dye per mol of silver halide for the emulsion which had small grains, after which the emulsion mixture was sulfur sensitized. This emulsion mixture was mixed with the aforementioned emulsified dispersion to prepare the first layer coating liquid of which the composition is indicated below.

The coating liquids for the second to the seventh layers were prepared using the same procedure as for the first layer coating liquid. 1-Oxy-3,5-dichloro-s-triazine sodium salt was used as a gelatin hardening agent for each layer.

The spectrally sensitizing dyes indicated below were used for each layer.

Blue-Sensitive Emulsion Layer

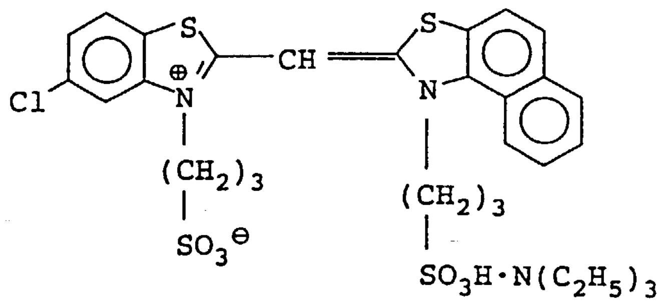

and

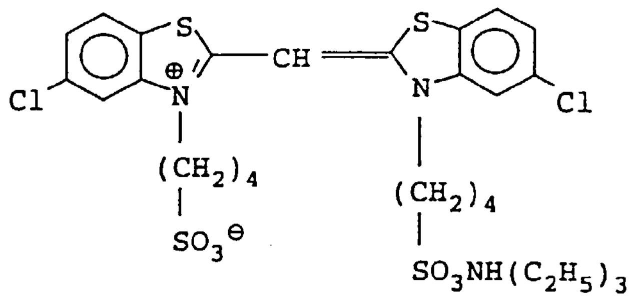

($2.0\times10^{-4}$ mol of each dye per mol of silver halide for the large grain size emulsion and $2.5\times10^{-4}$ mol of each dye per mol of silver halide for the small grain size emulsion)

Green-Sensitive Emulsion Layer

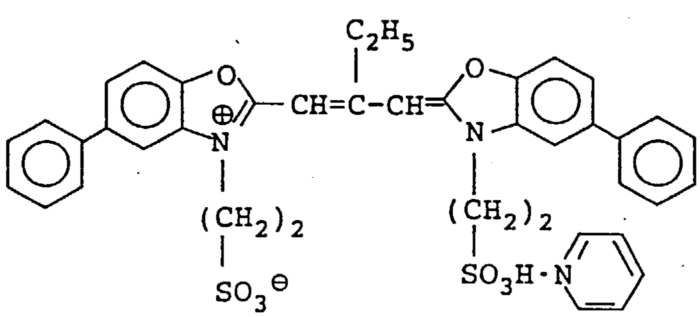

($4.0\times10^{-4}$ mol per mol of silver halide for the large grain size emulsion and $5.6\times10^{-4}$ mol per mol of silver halide for the small grain size emulsion) and

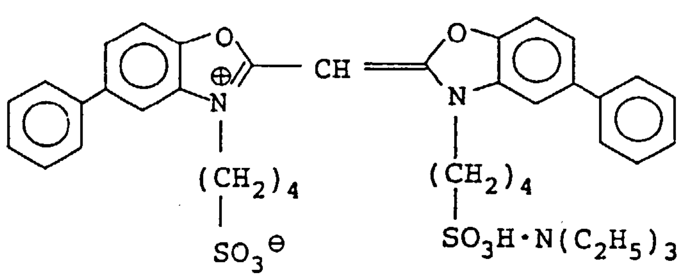

($7.0\times10^{-5}$ mol per mol of silver halide for the large grain size emulsion and $1.0\times10^{-5}$ mol per mol of silver halide for the small grain size emulsion)

Red Sensitive Emulsion Layer

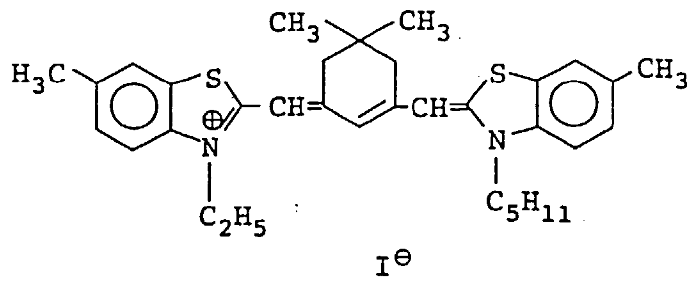

($0.9\times10^{-4}$ mol per mol of silver halide for the large grain size emulsion and $1.1\times10^{-4}$ mol per mol of silver halide for the small grain size emulsion)

The compound indicated below was added in an amount of $2.6\times10^{-3}$ mol per mol of silver halide to the red sensitive emulsion layer.

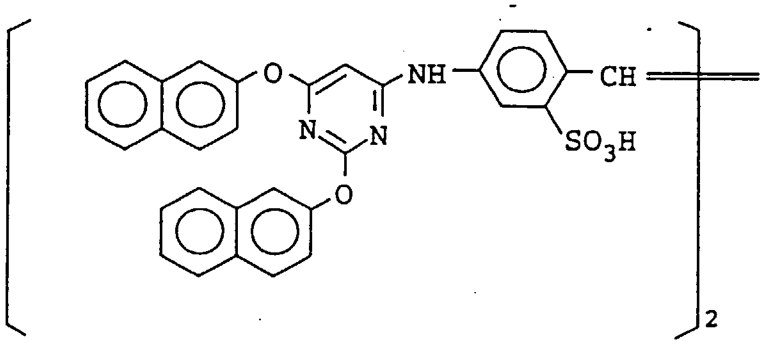

Furthermore, 1-(5-methylureidophenyl)-5-mercaptotetrazole was added to the blue, green and red sensitive emulsion layers in amounts, per mol of silver halide, of $8.5\times10^{-5}$ mol, $7.7\times10^{-4}$ mol and $2.5\times10^{-4}$ mol respectively.

Furthermore, 4-hydroxy-6-methyl-1,3,3a,7-tetra-azaindene was added to the blue and green sensitive emulsion layers in amounts, per mol of silver halide, of $1\times10^{-4}$ mol and $2\times10^{-4}$ mol respectively.

The dyes indicated below were added to the emulsion layers for anti-irradiation purposes.

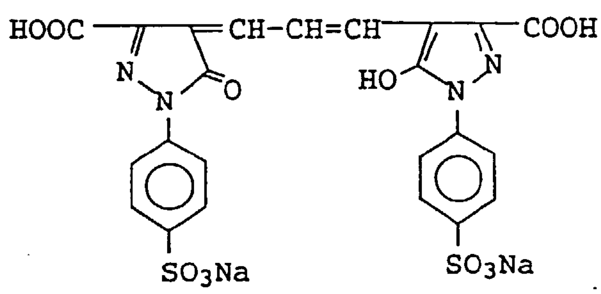

and

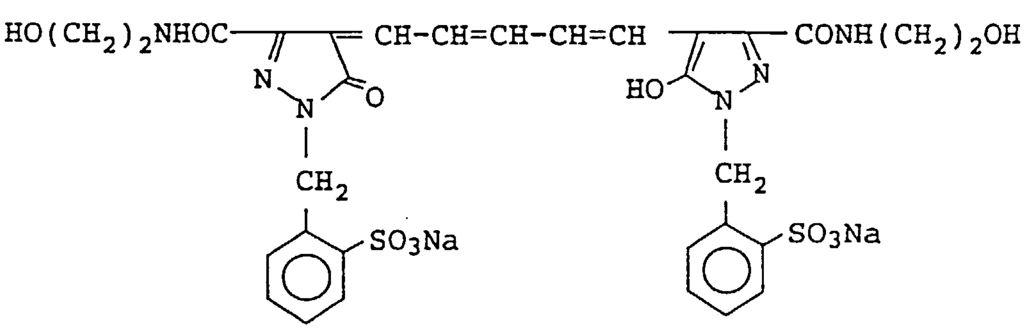

Layer Structure For Photosensitive Material Sample A

The composition of each layer was as indicated below. The numerical values indicate coated weights ($g/m^2$). In the case of silver halide emulsions the coated weight is shown as the calculated coated weight of silver.

Support

Polyethylene laminated paper
[white pigment ($TiO_2$) and blue dye (ultramarine) included in the polyethylene on the first layer side]

First Layer (Blue Sensitive Layer)

| | |
|---|---|
| The aforementioned silver chlorobromide emulsion | 0.30 |
| Gelatin | 1.86 |
| Yellow coupler (ExY) | 0.82 |
| Color image stabilizer (Cpd-1) | 0.19 |
| Solvent (Solv-1) | 0.35 |
| Color image stabilizer (Cpd-7) | 0.06 |

Second Layer (Anti-color Mixing Layer)

| | |
|---|---|
| Gelatin | 0.99 |
| Anti-color mixing agent (Cpd-5) | 0.08 |
| Solvent (Solv-1) | 0.16 |
| Solvent (Solv-3) | 0.02 |
| Solvent (Solv-4) | 0.08 |

Third Layer (Green Sensitive Layer)

| | |
|---|---|
| Silver chlorobromide emulsion (a 1:3 (silver mol ratio) mixture of cubic emulsions of average grain size 0.55 µm and 0.39 µm; the variation coefficients of the grain size distributions were 0.10 and 0.08, and each emulsion had 0.8 mol% AgBr included locally on the grain surfaces) | 0.12 |
| Gelatin | 1.24 |
| Magenta coupler (R-1) | 0.23 mol |
| Color image stabilizer (Cpd-3) | 0.15 |
| Color image stabilizer (Cpd-4) | 0.02 |
| Color image stabilizer (Cpd-9) | 0.02 |
| Solvent (Solv-2) | 0.40 |

Fourth Layer (Ultraviolet Absorbing Layer)

| | |
|---|---|
| Gelatin | 1.58 |
| Ultraviolet absorber (UV-1) | 0.47 |
| Anti-color mixing agent (Cpd-5) | 0.05 |
| Solvent (Solv-5) | 0.24 |

Fifth Layer (Red Sensitive Layer)

| | |
|---|---|
| Silver chlorobromide emulsion (a 1:4 (silver mol ratio) mixture of cubic emulsions of average grain size 0.58 μm and 0.45 μm; the variation coefficients of the grain size distributions were 0.09 and 0.11, and each emulsion had 0.6 mol% AgBr included locally on the grain surfaces) | 0.23 |
| Gelatin | 1.34 |
| Cyan coupler (ExC) | 0.32 |
| Color image stabilizer (Cpd-6) | 0.17 |
| Color image stabilizer (Cpd-7) | 0.40 |
| Color image stabilizer (Cpd-8) | 0.04 |
| Solvent (Solv-6) | 0.15 |

Sixth Layer (Ultraviolet Absorbing Layer)

| | |
|---|---|
| Gelatin | 0.53 |
| Ultraviolet absorber (UV-1) | 0.16 |
| Anti-color mixing agent (Cpd-5) | 0.02 |
| Solvent (Solv-5) | 0.08 |

Seventh Layer (Protective Layer)

| | |
|---|---|
| Gelatin | 1.33 |
| Acrylic modified poly(vinyl alcohol) (17% modification) | 0.17 |
| Liquid paraffin | 0.03 |

(ExY) Yellow Coupler

A 1 : 1 (mol ratio) mixture of:

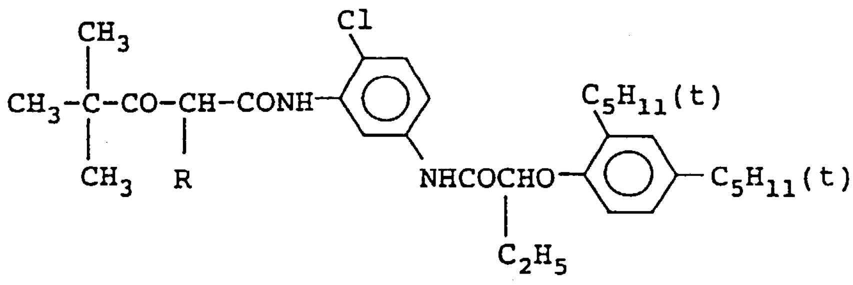

R= 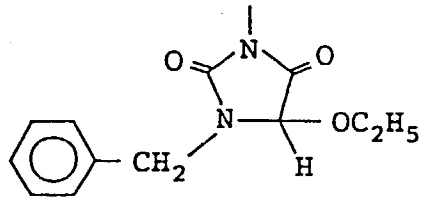

and

R= 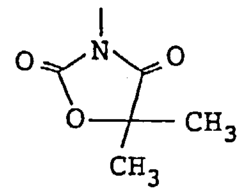

(ExC) Cyan Coupler

A 2 : 4 : 4 (by weight) mixture of:

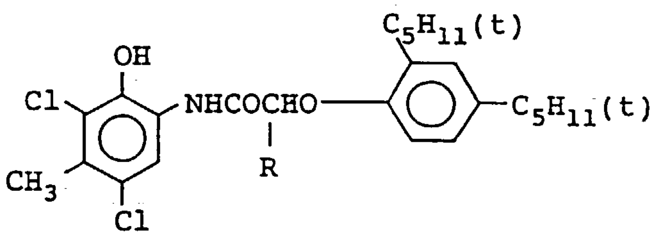

R = $C_2H_5$ and $C_4H_9$, and

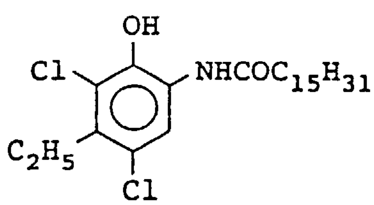

(Cpd-1) Color Image Stabilizer

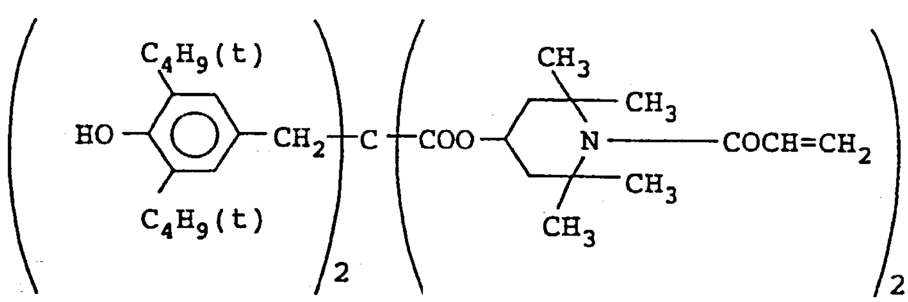

(Cpd-3) Color Image Stabilizer

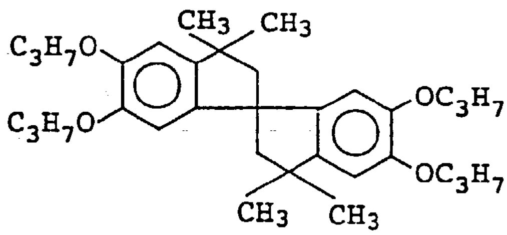

(Cpd-4) Color Image Stabilizer

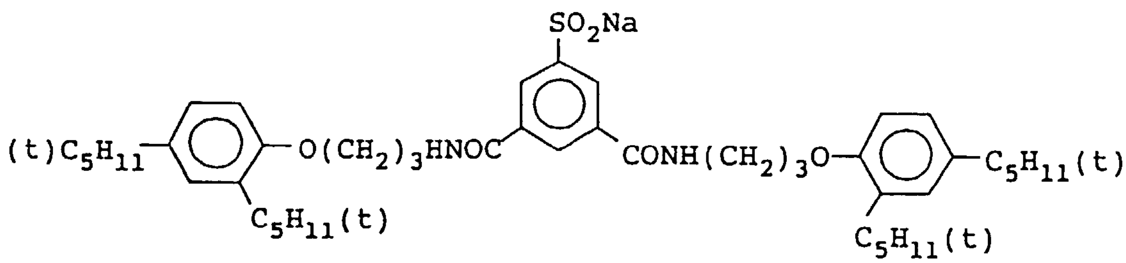

(Cpd-5) Anti-color Mixing Agent

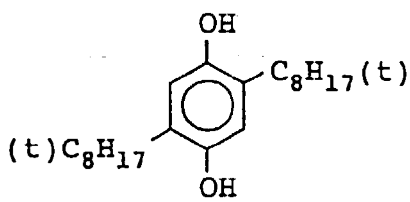

(Cpd-6) Color Image Stabilizer

A 2 : 4 : 4 (by weight) mixture of:

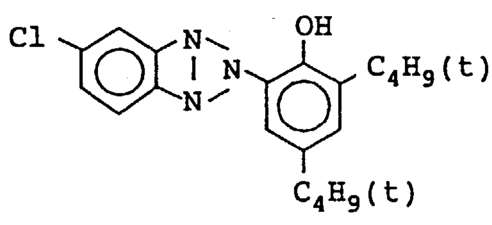

and

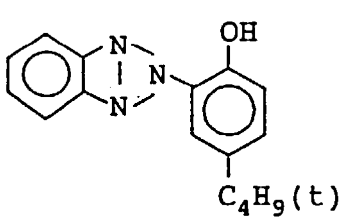

and

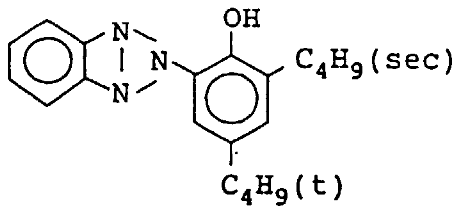

(Cpd-7) Color Image Stabilizer

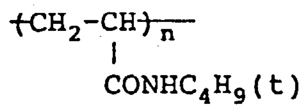

(average molecular weight: 60,000)

(Cpd-8) Color Image Stabilizer

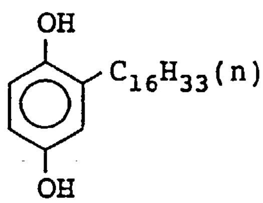

(Cpd-9) Color Image Stabilizer

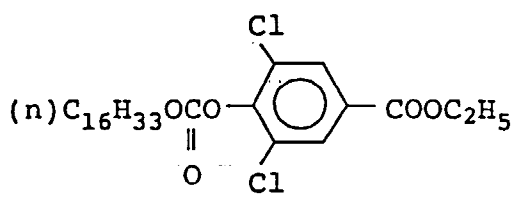

(UV-1) Ultraviolet Absorber

A 4 : 2 : 4 (by weight) mixture of:

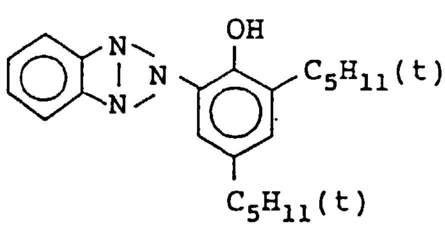

and

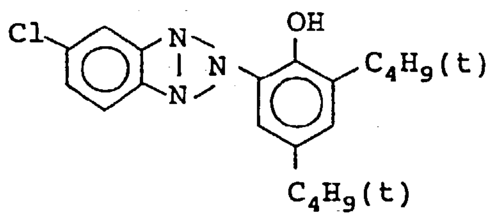

and

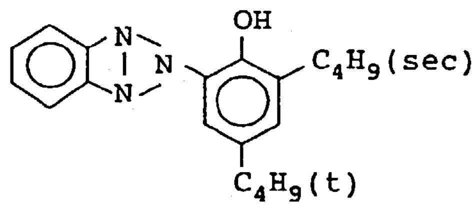

(Solv-1) Solvent

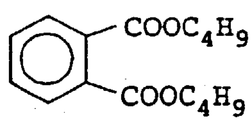

(Solv-2) Solvent

A 2 : 1 (by volume) mixture of:

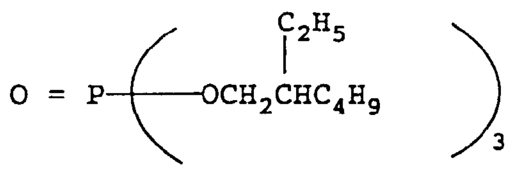

and

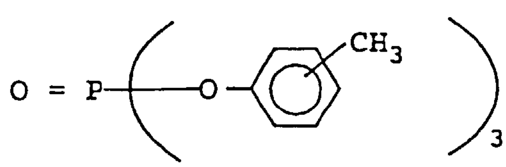

(Solv-3) Solvent

$O{=}P[OC_9H_{19}(iso)]_3$

(Solv-4) Solvent

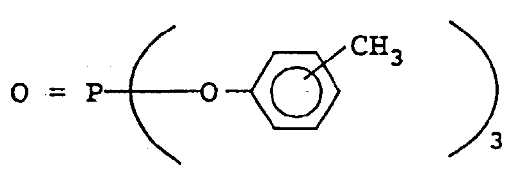

(Solv-5) Solvent

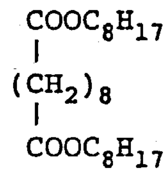

(Solv-6) Solvent

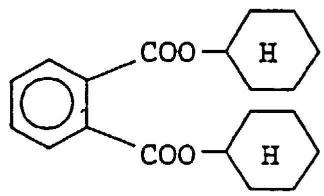

Next, Samples B to P were prepared in the same way as Sample A above except that the magenta coupler R1 in the third layer (green sensitive layer) of the photosensitive material Sample A described above was replaced by the magenta couplers of the present invention as shown in Table 2 below.

Next, each sample was subjected to an imagewise exposure through an optical wedge. The exposed samples were then processed continuously in a paper processor using the processing operations indicated below until replenishment had been carried out to the extent of twice the color development tank volume as a running test.

| Processing Operation | Temperature | Time | Replenishment Rate* | Tank Capacity |
|---|---|---|---|---|
| | (°C) | (sec) | (ml) | (ℓ) |
| Color Development | 35 | 45 | 80 | 17 |
| Bleach-fix | 30-35 | 45 | 215 | 17 |
| Rinse (1) | 30-35 | 20 | - | 10 |
| Rinse (2) | 30-35 | 20 | - | 10 |
| Rinse (3) | 30-35 | 20 | 350 | 10 |
| Drying | 70-80 | 60 | | |

*: Replenishment rate per square meter of photosensitive material

(A three tank counter flow system from Rinse (3) → Rinse (2) → Rinse (1) was used)

The composition of each processing bath was as indicated below.

| Color Development Bath | Tank Solution | Replenisher |
|---|---|---|
| Water | 800 ml | 800 ml |
| Ethylenediamine-N,N,N,N-tetramethylenephosphonic acid | 1.5 g | 2.0 g |
| Triethanolamine | 8.0 g | 12.0 g |
| Sodium chloride | 1.4 g | - |
| Potassium carbonate | 25 g | 25 g |
| N-Ethyl-N-(β-methanesulfonamidoethyl)-3-methyl-4-aminoaniline sulfate | 5.0 g | 7.0 g |
| N,N-Bis(carboxymethyl)hydrazine | 5.5 g | 7.0 g |
| Fluorescent whitener (WHITEX 4B) | 1.0 g | 2.0 g |
| Water | to make up to 1000 ml | 1000 ml |
| pH (25°C) | 10.05 | 10.45 |

Bleach-Fix Bath (Tank Solution = Replenisher)

| | |
|---|---|
| Water | 400 ml |
| Ammonium thiosulfate (700 g/ℓ)) | 100 ml |
| Sodium sulfite | 17 g |
| Ethylenediaminetetraacetic acid ferric ammonium salt | 55 g |
| Ethylenediaminetetraacetic acid disodium salt | 5 g |
| Ammonium bromide | 40 g |
| Water | to make up to 1000 ml |
| pH (25°C) | 6.0 |

Rinse Bath (Tank Solution = Replenisher)

Deionized water (calcium and magnesium both not more than 3 ppm)

TABLE 2

| Photosensitive Material | Magenta Coupler | Change in Photographic Properties: $\Delta D_{min}$ | $\Delta D_{max}$ | Δ Gradation | Δ Staining | Remarks |
|---|---|---|---|---|---|---|
| A | R-1 | +0.06 | -0.30 | -0.10 | +0.24 | Comparative Example |
| B | R-2 | +0.06 | -0.32 | -0.11 | +0.22 | " |
| C | R-3 | +0.07 | -0.31 | -0.11 | +0.21 | " |
| D | R-4 | +0.06 | -0.33 | -0.14 | +0.22 | " |
| E | R-5 | +0.07 | -0.31 | -0.13 | +0.21 | " |
| N | m-1 | +0.03 | -0.06 | -0.07 | +0.10 | Present Invention |
| O | m-2 | +0.02 | -0.06 | -0.07 | +0.10 | " |
| P | m-3 | +0.03 | -0.07 | -0.08 | +0.12 | " |

(R-1)

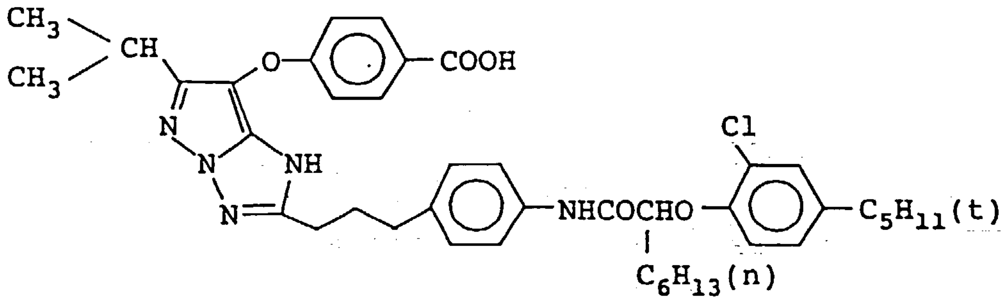

(Compound disclosed in JP-A-61-53644)

(R-2)

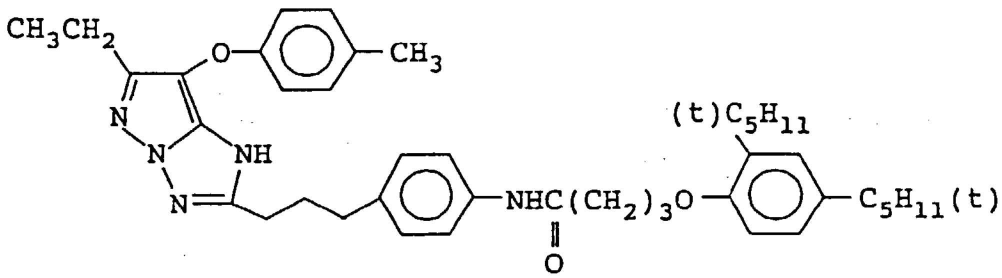

(Compound disclosed in JP-A-61-53644)

(R-3)

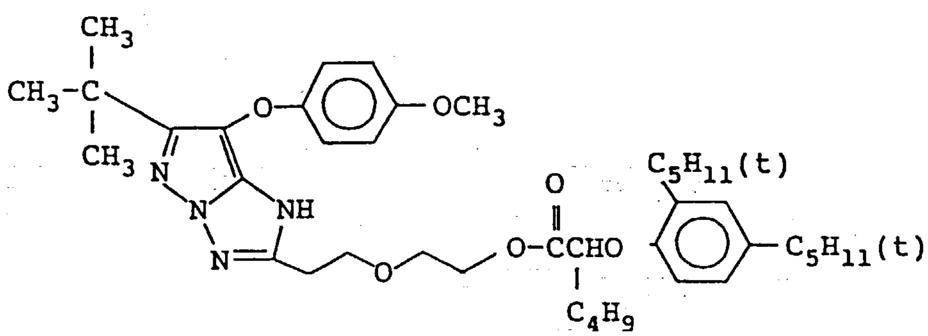

(Compound disclosed in JP-A-61-53644)

(R-4)

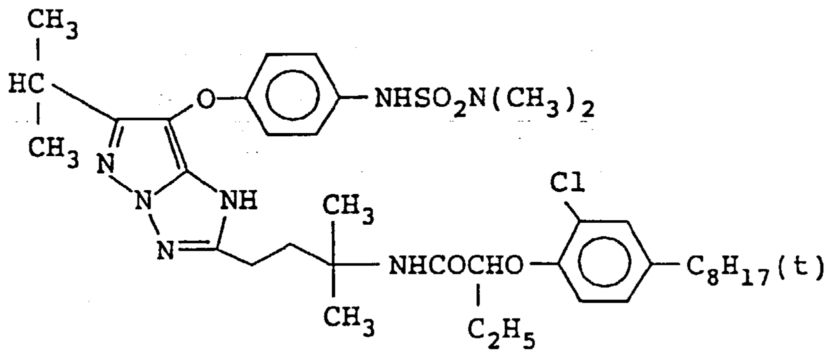

(Compound disclosed in EP-A- 177,765)

(R-5)

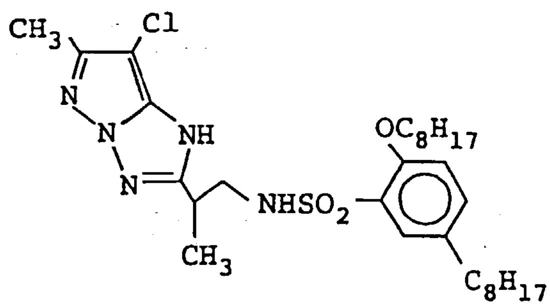

Photographic properties were investigated in terms of the changes in $D_{min}$ (minimum density) of the magenta density, $D_{max}$ (maximum density) of the magenta layer density, gradation and the extent of post-processing staining test. Post-processing staining was determined by measuring the change in magenta reflection density value of a non-image part as observed immediately after processing and that observed after standing for 30 days under conditions of 60°C. 70% RH, and the results obtained are shown in Table 2.

It is clear from Table 2 that when a magenta coupler of the present invention is used, the change or deviation in photographic properties before and after running is suppressed to a remarkable degree in comparison to the photosensitive materials containing the magenta couplers of the prior art.

Furthermore, when the magenta couplers of the present invention are used, the occurrence of staining due to ageing after processing is suppressed to a remarkable degree.

Moreover, when a magenta coupler of the present invention is used the half value width is narrow and good color reproduction is observed.

EXAMPLE 3

A color photographic photosensitive material was prepared by the lamination coating of the first to the twelfth layers indicated below on a paper support which had been laminated on both sides with polyethylene. Titanium white as a white pigment and a trace of ultramarine as a blue dye were included in the polyethylene on the first layer side of the support.

Photosensitive Layer Composition

The components and coated weights in units of $g/m^2$ are indicated below. In the case of silver halides the coated weights are indicated after calculation as silver.

First Layer (Gelatin Layer)

| | |
|---|---|
| Gelatin | 1.30 |

Second Layer (Anti-halation Layer)

| | |
|---|---|
| Black colloidal silver | 0.10 |
| Gelatin | 0.70 |

Third Layer (Low Speed Red Sensitive Layer)

| | |
|---|---|
| Silver chloroiodobromide (1 mol% AgCl, 4 mol% AgI, average grain size 0.3 µm size distribution 10%, cubic grains, core iodide type core/shell) spectrally sensitized with the red sensitizing dyes (ExS-1, 2, 3) | 0.06 |
| Silver iodobromide (5 mol% AgI, average grain size 0.45 µm size distribution 20%, tabular (aspect ratio = 5)) spectrally sensitized with the red sensitizing dyes (ExS-1, 2, 3) | 0.10 |
| Gelatin | 1.00 |
| Cyan coupler (ExC-1) | 0.14 |
| Cyan coupler (ExC-2) | 0.07 |
| Anti-color mixing agent (equal amounts of Cpd-2, 3, 4, 13) | 0.12 |
| Coupler dispersion medium (Cpd-5) | 0.03 |
| Coupler solvent (equal amounts of Solv-1, 2, 3) | 0.06 |

Fourth Layer (High Speed Red Sensitive Layer)

| | |
|---|---|
| Silver iodobromide (6 mol% AgI, average grain size 0.75 µm size distribution 25%, tabular grains (aspect ratio = 8, core iodide)) spectrally sensitized with the red sensitizing dyes (ExS-1, 2, 3) | 0.15 |
| Gelatin | 1.00 |
| Cyan coupler (ExC-1) | 0.20 |
| Cyan coupler (ExC-2) | 0.10 |
| Anti-color mixing agent (equal amounts of Cpd-2, 3, 4, 13) | 0.15 |
| Coupler dispersion medium (Cpd-5) | 0.03 |
| Coupler solvent (equal amounts of Solv-1, 2, 3) | 0.10 |

Fifth Layer (Interlayer)

| | |
|---|---|
| Magenta colloidal silver | 0.02 |

(continued)

| | |
|---|---|
| Gelatin | 1.00 |
| Anti-color mixing agent (Cpd-6, 7) | 0.08 |
| Anti-color mixing agent solvent (Solv-4, 5) | 0.16 |
| Polymer latex (Cpd-8) | 0.10 |

Sixth Layer (Low Speed Green Sensitive Layer)

| | |
|---|---|
| Silver chloroiodobromide (1 mol% AgCl, 2.5 mol% AgI, average grain size 0.28 µm grain size distribution 12%, cubic grains, core iodide type core/shell) spectrally sensitized with the green sensitizing dyes (ExS-4) | 0.04 |
| Silver iodobromide (2.8 mol% AgI, average grain size 0.45 µm grain size distribution 12%, tabular (aspect ratio = 5)) spectrally sensitized with the green sensitizing dyes (ExS-4) | 0.06 |
| Gelatin | 0.80 |
| Magenta coupler (see table 3) | 0.12 mmol |
| Anti-color mixing agent (Cpd-9) | 0.10 |
| Anti-staining agent (equal amount of Cpd-10, 22) | 0.01 |
| Anti-staining agent (Cpd-11) | 0.001 |
| Anti-staining agent (Cpd-12) | 0.01 |
| Coupler dispersion medium (Cpd-5) | 0.05 |
| Coupler solvent (Solv-4, 6) | 0.15 |

Seventh Layer (High Speed Green Sensitive Layer)

| | |
|---|---|
| Silver iodobromide (3.5 mol% AgI, average grain size 0.9 µm grain size distribution 23%, tabular (aspect ratio = 9, uniform iodide)) spectrally sensitized with the green sensitizing dyes (ExS-4) | 0.10 |
| Gelatin | 0.80 |
| Magenta coupler (See Table 3) | 0.12 mmol |
| Anti-color mixing agent (Cpd-9) | 0.10 |
| Anti-staining agent (equal amount of Cpd-10, 22) | 0.01 |
| Anti-staining agent (Cpd-11) | 0.001 |

(continued)

| | |
|---|---|
| Anti-staining agent (Cpd-12) | 0.01 |
| Coupler dispersion medium (Cpd-5) | 0.05 |
| Coupler solvent (Solv-4, 6) | 0.15 |

Eighth Layer (Yellow Filter Layer)

| | |
|---|---|
| Yellow colloidal silver | 0.20 |
| Gelatin | 1.00 |
| Anti-color mixing agent (Cpd-7) | 0.06 |
| Anti-color mixing agent solvent (Solv-4, 5) | 0.15 |
| Polymer latex (Cpd-8) | 0.10 |

Ninth Layer (Low Speed Blue Sensitive Layer)

| | |
|---|---|
| Silver chloroiodobromide (2 mol% AgCl, 2.5 mol% AgI, average grain size 0.35 μm grain size distribution 8%, cubic grains, core iodide type core/shell) spectrally sensitized with the blue sensitizing dyes (ExS-5, 6) | 0.07 |
| Silver iodobromide (2.5 mol% AgI, average grain size 0.45 μm grain size distribution 16%, tabular (aspect ratio = 6)) spectrally sensitized with the blue sensitizing dyes (ExS-5, 6) | 0.10 |
| Gelatin | 0.50 |
| Yellow coupler (ExY-1) | 0.20 |
| Anti-staining agent (Cpd-11) | 0.001 |
| Anti-color mixing agent (Cpd-6) | 0.10 |
| Coupler dispersion medium (Cpd-5) | 0.05 |
| Coupler solvent (Solv-2) | 0.05 |

Tenth Layer (High Speed Blue Sensitive Layer)

| | |
|---|---|
| Silver iodobromide (2.5 mol% AgI, average grain size 1.2 μm grain size distribution 21%, tabular (aspect ratio = 14)) spectrally sensitized with the blue sensitizing dyes (ExS-5, 6) | 0.25 |
| Gelatin | 1.00 |
| Yellow coupler (ExY-1) | 0.40 |
| Anti-staining agent (Cpd-11) | 0.002 |
| Anti-color mixing agent (Cpd-6) | 0.10 |
| Coupler dispersion medium (Cpd-5) | 0.05 |
| Coupler solvent (Solv-2) | 0.10 |

Eleventh Layer (Ultraviolet Absorbing Layer)

| | |
|---|---|
| Gelatin | 1.50 |
| Ultraviolet absorber (Cpd-1, 3, 13) | 1.00 |
| Anti-color mixing agent (Cpd-6, 14) | 0.06 |
| Dispersion medium (Cpd-5) | |
| Ultraviolet absorber solvent (Solv-1, 2) | 0.15 |
| Anti-irradiation dye (Cpd-15, 16) | 0.02 |
| Anti-irradiation dye (Cpd-17, 18) | 0.02 |

Twelfth Layer (Protective Layer)

| | |
|---|---|
| Fine grained silver chlorobromide (97 mol% AgCl, average grain size 0.2 μ) | 0.07 |
| Modified Poval (Ethenol polymer) | 0.02 |
| Gelatin | 1.50 |
| Gelatin hardening agent (H-1) | 0.17 |

Moreover, "Alkanol XC" (DuPont Co.) and sodium alkylbenzenesulfonate were used as emulsification and dispersion promotors, and succinic acid ester and "Magefac F-120" (Dainippon Ink and Chemicals, Inc.) were used as coating promotors in each layer. The compounds (Cpd-19, 20, 21) were used as stabilizers in the layers which contained silver halides or colloidal silver. The compounds used in this Example are indicated below.

ExS-1

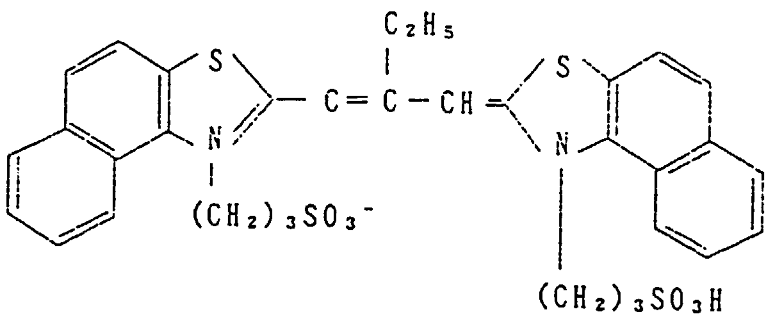

ExS-2

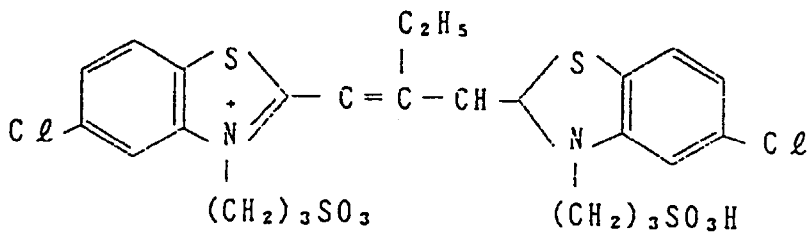

ExS-3

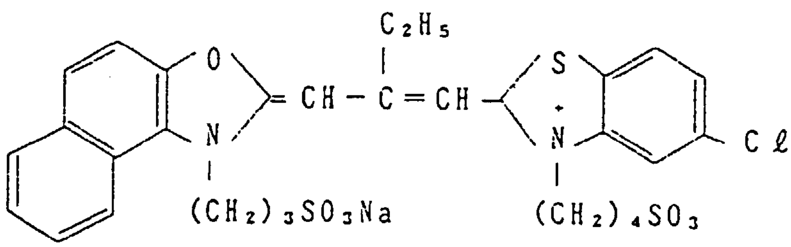

ExS-4

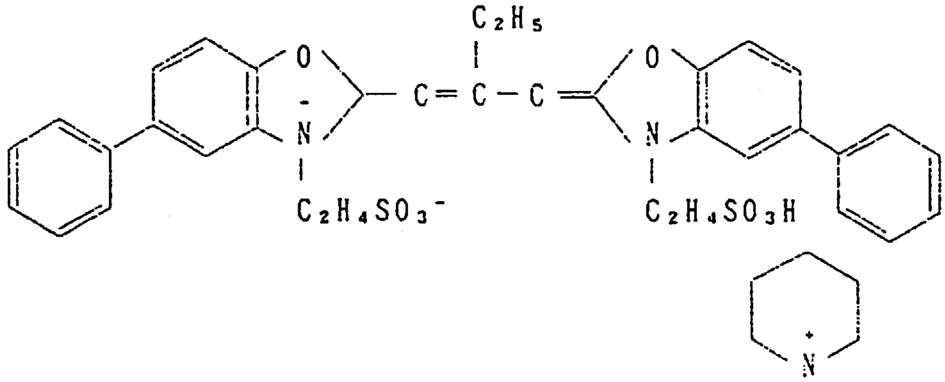

ExS-5

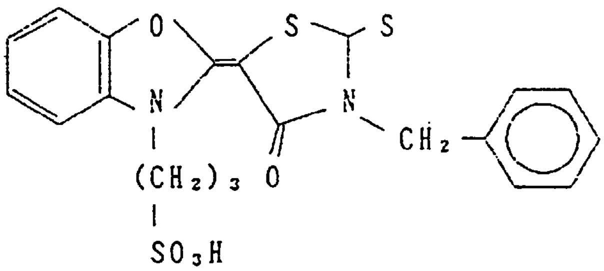

ExS-6

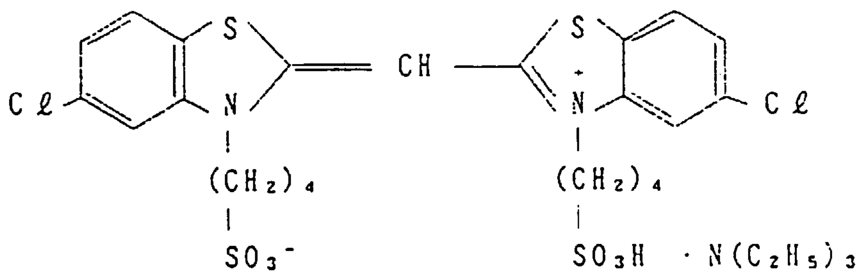

Cpd-1

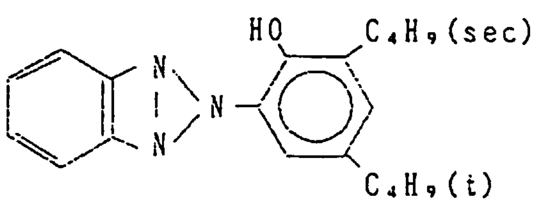

Cpd-2

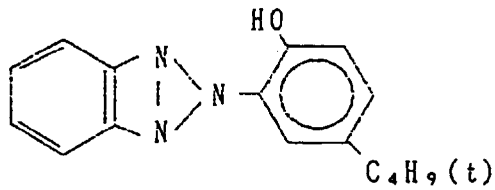

Cpd-3

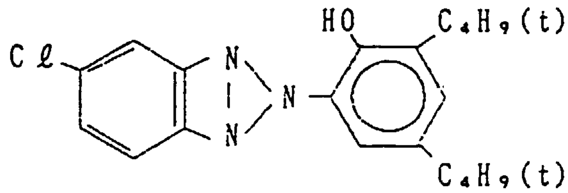

Cpd-4

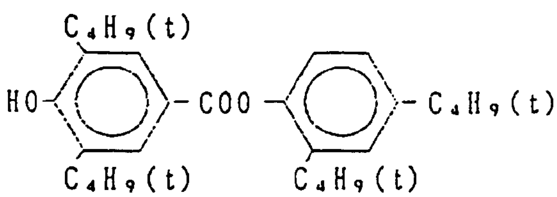

Cpd-5

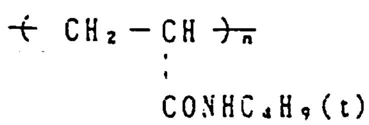

(n = 100 ~ 1000)

Cpd-6

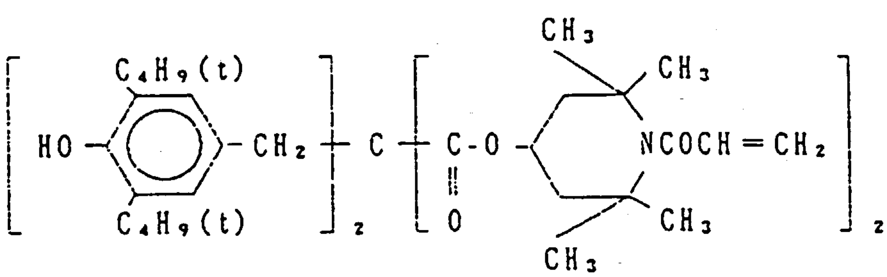

Cpd-7

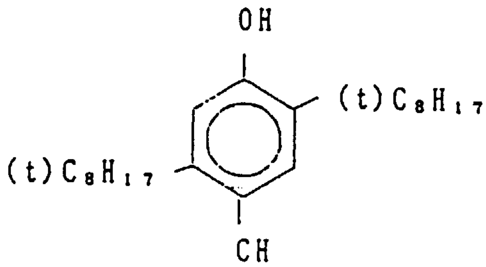

Cpd-8 Poly(ethyl acrylate)

Cpd-9

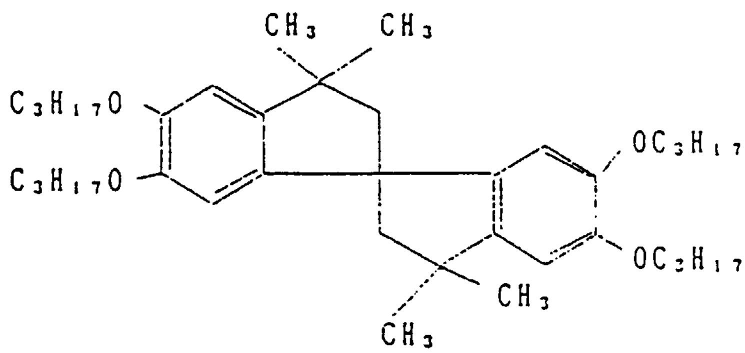

Cpd-10

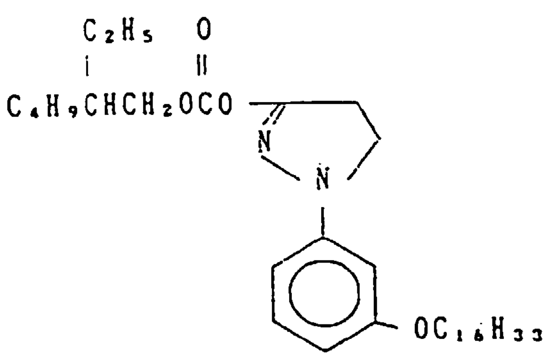

Cpd-11

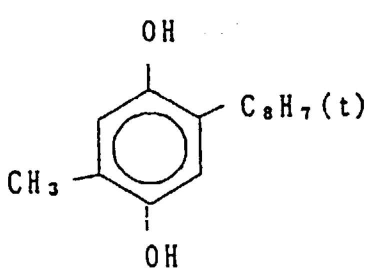

Cpd-12

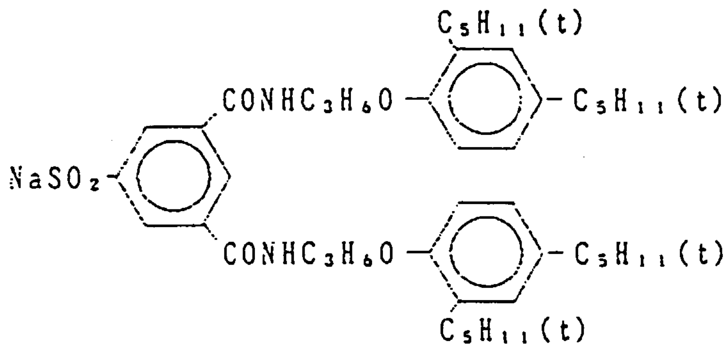

Cpd-13

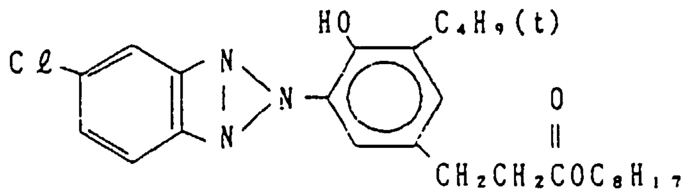

Cpd-14

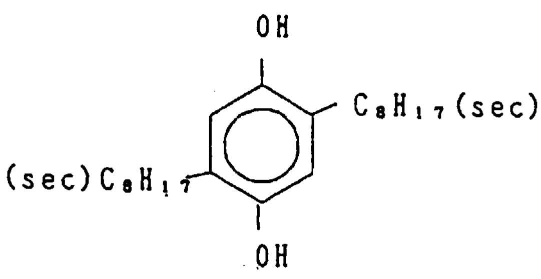

Cpd-15

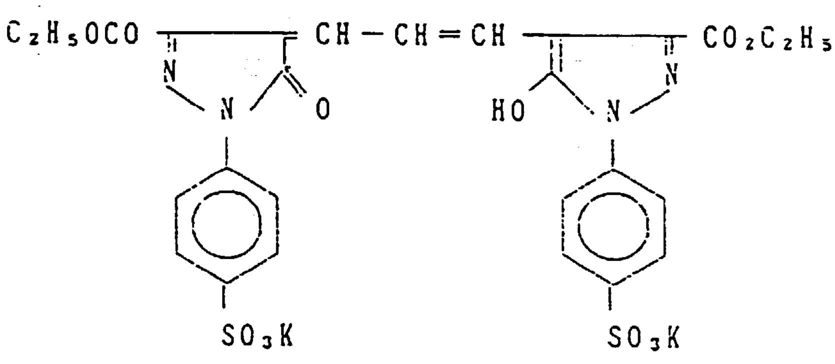

Cpd-16

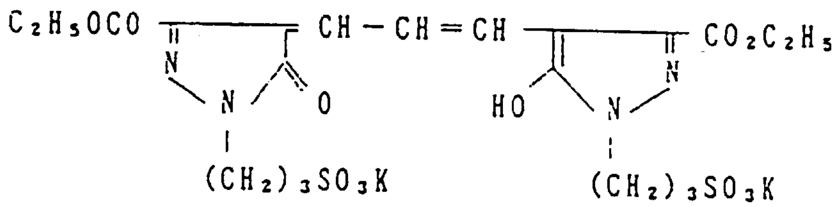

Cpd-17

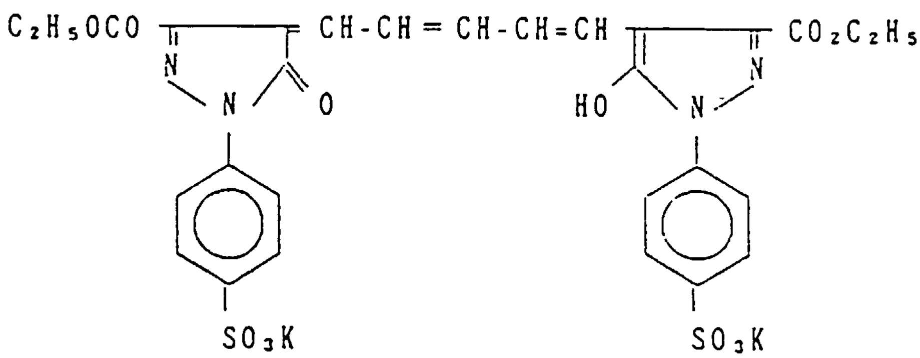

Cpd-18

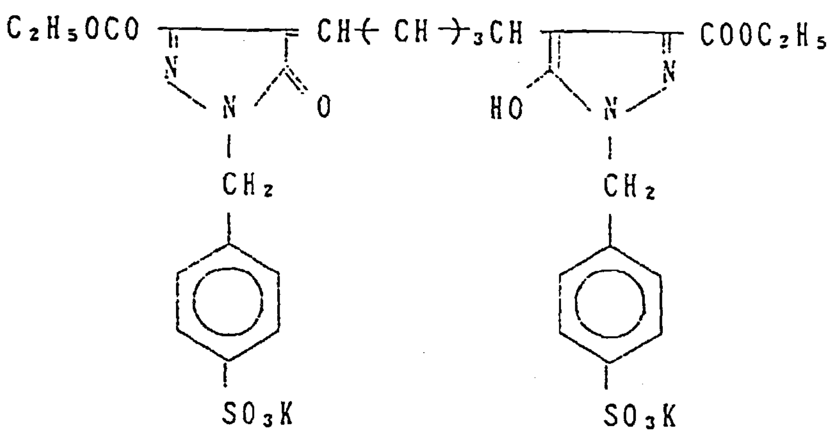

Cpd-19

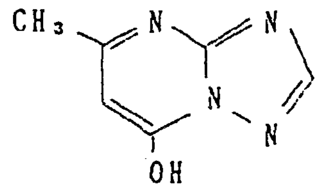

Cpd-20

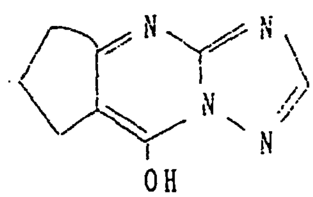

Cpd-21

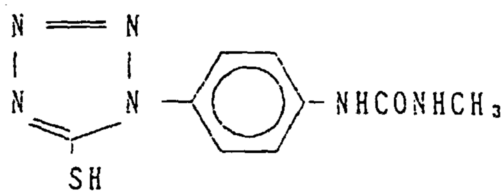

Cpd-22

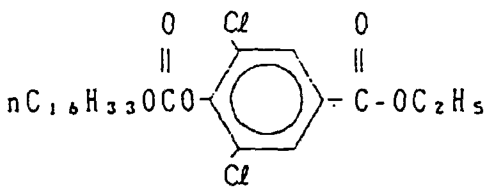

ExC-1

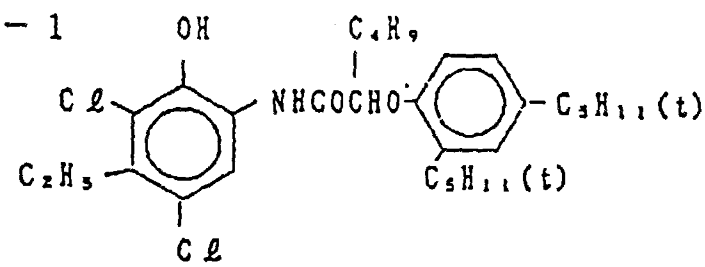

ExC-2

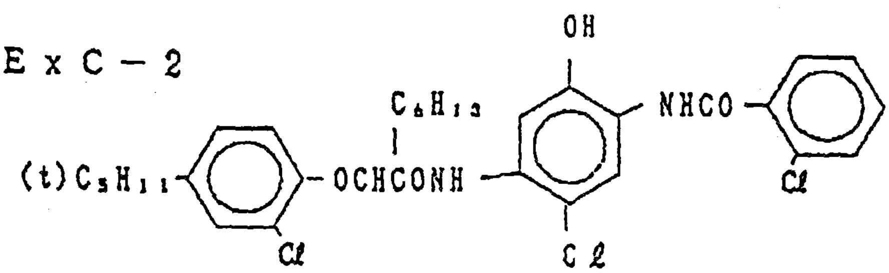

ExY-1

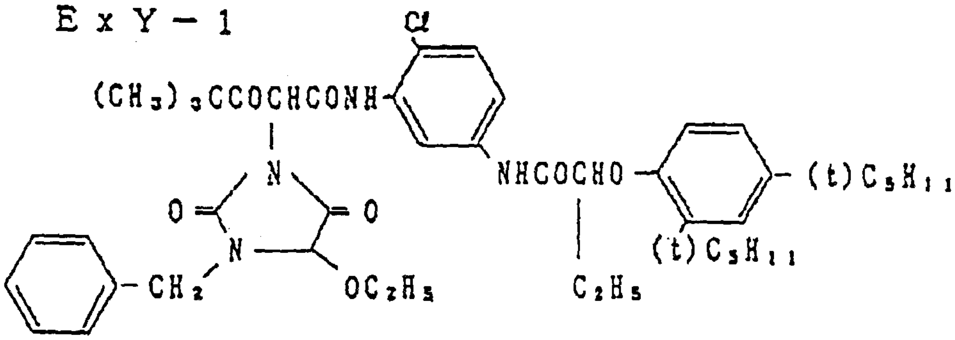

| | |
|---|---|
| Solv-1 | Di(2-ethylhexyl) phthalate |
| Solv-2 | Trinonyl phosphate |
| Solv-3 | Di(3-methylhexyl)phthalate |
| Solv-4 | Tricresyl phosphate |
| Solv-5 | Dibutyl phthalate |
| Solv-6 | Trioctyl phosphate |

H-1

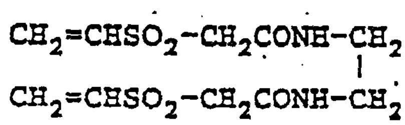

Other photosensitive materials were prepared in the same way except that the magenta coupler in the sixth and seventh layers was modified as shown in Table 3 below.

After exposing the silver halide color photographic materials which had been prepared in the way described above they were processed in accordance with the processing operations indicated below.

| Processing Operation | Time | Temperature |
|---|---|---|
| First Development | 6 minutes | 38°C |
| Water Wash | 2 minutes | 38°C |
| Reversal | 2 minutes | 38°C |
| Color Development | 6 minutes | 38°C |
| Conditioning | 2 minutes | 38°C |
| Bleaching | 6 minutes | 38°C |
| Fixing | 4 minutes | 38°C |
| Water Wash | 4 minutes | 38°C |
| Stabilization | 1 minute | 25°C |

The composition of each processing bath was as indicated below.

First Development Bath

| | |
|---|---|
| Nitrilo-N,N,N-trimethylenephosphonic acid pentasodium salt | 2.0 grams |
| Sodium sulfite | (See Table 3) |
| Hydroquinone monosulfonic acid potassium salt | 20 grams |
| Potassium carbonate | 33 grams |
| 1-Phenyl-4-methyl-4-hydroxymethyl-3-pyrazolidone | 2.0 grams |
| Potassium bromide | 2.5 grams |
| Potassium thiocyanate | 1.2 grams |
| Potassium iodide | 2.0 mg |
| Water | to make up to 1000 ml |
| pH | 9.60 |

The pH was adjusted with hydrochloric acid or potassium hydroxide.

Reversal Bath

| | |
|---|---|
| Nitrilo-N,N,N-trimethylenephosphonic acid pentasodium salt | 3.0 grams |
| Stannous chloride dihydrate | 1.0 gram |
| p-Aminophenol | 0.1 gram |
| Sodium hydroxide | 8 grams |
| Glacial acetic acid | 15 ml |
| Water | to make up to 1000 ml |
| pH | 6.00 |

The pH was adjusted with hydrochloric acid or sodium hydroxide.

Color Development Bath

| | |
|---|---|
| Nitrilo-N,N,N-trimethylenephosphonic acid pentasodium salt | 2.0 grams |
| Sodium sulfite | 7.0 grams |

| | |
|---|---|
| Trisodium phosphate dodecahydrate | 36 grams |
| Potassium bromide | 1.0 gram |
| Potassium iodide | 90 mg |
| Sodium hydroxide | 3.0 grams |
| Citrazinic acid | 1.5 grams |
| N-Ethyl-N-(β-methanesulfonamidoethyl)-3-methyl-4-aminoaniline sulfate | 11 grams |
| 3,6-Dithiaoctane-1,8-diol | 1.0 gram |
| Water | to make up to 1000 ml |
| pH | 11.80 |

The pH was adjusted with hydrochloric acid or potassium hydroxide.

Conditioner

| | |
|---|---|
| Ethylenediaminetetraacetic acid, di-sodium salt dihydrate | 8.0 grams |
| Sodium sulfite | 12 grams |
| 1-Thioglycerine | 0.4 ml |
| Water | to make up to 1000 ml |
| pH | 6.20 |

The pH was adjusted with hydrochloric acid or sodium hydroxide.

Bleach Bath

| | |
|---|---|
| Ethylenediaminetetraacetic acid disodium salt dihydrate | 2.0 grams |
| Ethylenediaminetetraacetic acid ferric ammonium salt dihydrate | 120 grams |
| Potassium bromide | 100 grams |
| Ammonium nitrate | 10 grams |
| Water | to make up to 1000 ml |
| pH | 5.70 |

The pH was adjusted using hydrochloric acid or sodium hydroxide.

Fixer Bath

| | |
|---|---|
| Ammonium thiosulfate | 80 grams |
| Sodium sulfite | 5.0 grams |
| Sodium bisulfite | 5.0 grams |
| Water | to make up to 1000 ml |
| pH | 6.60 |

The pH was adjusted using hydrochloric acid or aqueous ammonia

Stabilizer

| | |
|---|---|
| Formalin (37%) | 5.0 ml |
| Polyoxyethylene p-mononoylphenyl ether (average degree of polymerization 10) | 0.5 ml |
| Water | to make up to 1000 ml |
| pH | Not Adjusted |

TABLE 3

| Photosensitive material | Magenta Coupler | Photographic Properties Sulfite Ion Concentration (mol/ℓ) 0.6x10$^{-2}$ | 1.2x10$^{-2}$ | 2/4x10$^{-2}$ | Remarks |
|---|---|---|---|---|---|
| A | R-1*1) | 2.31 | 2.00 | 1.82 | Comparative Example |
| B | R-2*1) | 2.34 | 2.10 | 1.91 | " |
| H | m-2 | 2.34 | 2.32 | 2.21 | Present Invention |
| I | m-3 | 2.36 | 2.33 | 2.20 | " |
| J | m-4 | 2.37 | 2.31 | 2.21 | " |
| K | m-18 | 2.36 | 2.32 | 2.20 | " |
| L | m-19 | 2.36 | 2.33 | 2.19 | " |
| M | m-24 | 2.37 | 2.31 | 2.18 | " |
| N | M-29 | 2.33 | 2.31 | 2.23 | " |

*1) Same as Example 2

As is clear from Table 3, when a magenta coupler of the present invention is used the change in photographic properties with fluctuations in the sulfite ion concentration is suppressed to a remarkable degree.

## Claims

1. A silver halide color photographic material comprising a support having thereon at least one silver halide emulsion layer containing at least one coupler which is represented by formula (II) as follows:

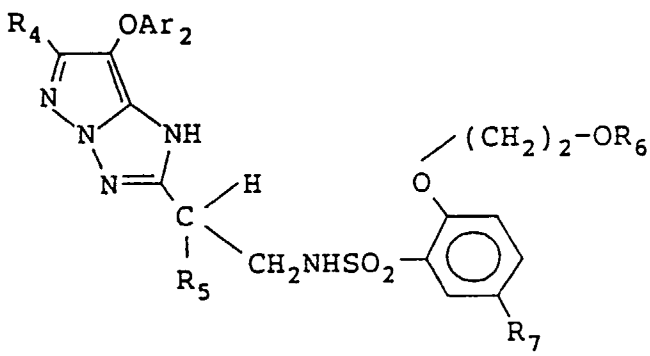

(II)

wherein $R_4$ and $R_5$ each represents an alkyl group; $R_6$ represents an alkyl group or an aryl group; $R_7$ represents a substituent group; and $Ar_2$ represents an aryl group.

2. A silver halide color photographic material as in claim 1, wherein the coupler represented by formula (II) is added in an amount of from 0.01 mmol to 2 mmol per square meter of said photosensitive material.

3. A silver halide color photographic material as in claim 1, wherein the coupler represented by formula (II) is added in an amount of from 0.1 mmol to 1.5 mmol per square meter of said photosensitive material.

4. A silver halide color potographic material as in claim 1, wherein the support contains thereon at least one blue light sensitive layer, at least one green light sensitive layer, and at least one red light sensitive layer.

5. A silver halide color photographic material as in claim 1, wherein $R_4$ and $R_5$ each represents a linear chain or branched chain alkyl group having from 1 to 35 carbon atoms; $R_6$ represents a substituted or unsubstituted, linear chain or branched chain alkyl group having at least 2 carbon atoms or a substituted or unsubstituted aryl group having at least 6 carbon atoms; $R_7$ represents a linear chain or branched chain alkyl group having at least 4 carbon atoms, an alkoxy group having at least 4 carbon atoms, an acylamino group having at least 5 carbons or an alkylsulfonamido or arylsulfonamido group having at least 6 carbon atoms; and $Ar_2$ represents a substituted or unsubstituted phenyl group or naphthyl group.

## Patentansprüche

1. Photographisches Silberhalogenidfarbmaterial, umfassend einen Träger mit wenigstens einer darauf angeordneten Silberhalogenidemulsionsschicht, enthaltend wenigstens einen Kuppler, dargestellt durch folgende Formel (II):

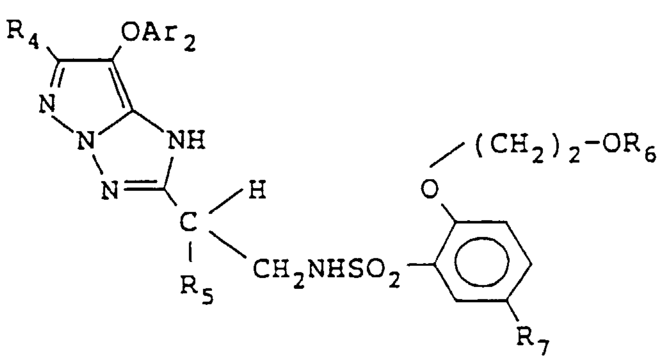

(II)

worin bedeuten:
$R_4$ und $R_5$ jeweils eine Alkylgruppe; $R_6$ eine Alkylgruppe oder eine Arylgruppe; $R_7$ eine Substituentengruppe; und $Ar_2$ eine Arylgruppe.

2. Photographisches Silberhalogenidfarbmaterial nach Anspruch 1, worin der durch Formel (II) dargestellte Kuppler in einer Menge von 0,01 mMol bis 2 mMol pro Quadratmeter des photoempfindlichen Materials zugegeben wird.

3. Photographisches Silberhalogenidfarbmaterial nach Anspruch 1, worin der durch Formel (II) dargestellte Kuppler in einer Menge von 0,1 mMol bis 1,5 mmol pro Quadratmeter des photoempfindlichen Materials zugegeben wird.

4. Photographisches Silberhalogenidfarbmaterial nach Anspruch 1, worin der Träger wenigstens eine blaulichtempfindliche Schicht, wenigstens eine grünlichtempfindliche Schicht und wenigstens eine rotlichtempfindliche Schicht enthält.

5. Photographisches Silberhalogenidfarbmaterial nach Anspruch 1, worin bedeuten:
$R_4$ und $R_5$ jeweils eine linearkettige oder verzweigtkettige Alkylgruppe mit 1 bis 35 Kohlenstoffatomen; $R_6$ eine substitutierte oder nicht-substituierte, linearkettige oder verzweigtkettige Alkylgruppe mit wenigstens 2 Kohlenstoffatomen oder eine substitutierte oder nicht-substituierte Arylgruppe mit wenigstens 6 Kohlenstoffatomen; $R_7$ eine linearkettige oder verzweigtkettige Alkylgruppe mit wenigstens 4 Kohlenstoffatomen, eine Alkoxygruppe mit wenigstens 4 Kohlenstoffatomen, eine Acylaminogruppe mit wenigstens 5 Kohlenstoffatomen oder eine Alkylsulfonamido- oder Arylsulfonamidogruppe mit wenigstens 6 Kohlenstoffatomen; und $Ar_2$ eine substituierte oder nicht-substituierte Phenyl- oder Naphthylgruppe.

**Revendications**

1. Un matériau photographique couleur à l'halogénure d'argent comprenant un support portant au moins une couche d'émulsion d'halogénure d'argent contenant au moins un coupleur qui est représenté par la formule (II) suivante :

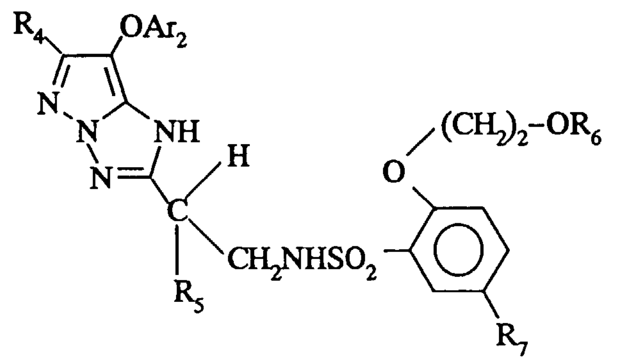

(II)

dans laquelle $R_4$ et $R_5$ représentent chacun un groupe alkyle ; $R_6$ représente un groupe alkyle ou un groupe aryle ; $R_7$ représente un substituant ; et $Ar_2$ représente un groupe aryle.

2. Un matériau photographique couleur à l'halogénure d'argent selon la revendication 1, dans lequel le coupleur représenté par la formule (II) est ajouté en quantité de 0,01 à 2 mmol/m$^2$ dudit matériau photosensible.

3. Un matériau photographique couleur à l'halogénure d'argent selon la revendication 1, dans lequel le coupleur représenté par la formule (II) est ajouté en quantité de 0,1 à 1,5 mmol/m$^2$ dudit matériau photosensible.

4. Un matériau photographique couleur à l'halogénure d'argent selon la revendication 1, dans lequel le support porte au moins une couche sensible à la lumière bleue, au moins une couche sensible à la lumière verte et au moins une couche sensible à la lumière rouge.

5. Un matériau photographique couleur à l'halogénure d'argent selon la revendication 1, dans lequel $R_4$ et $R_5$ représentent chacun un groupe alkyle à chaîne droite ou ramifiée en $C_1$-$C_{35}$ ; $R_6$ représente un groupe alkyle à chaîne droite ou ramifiée ayant au moins 2 atomes de carbone, substitué ou non ou un groupe aryle ayant au moins 6 atomes de carbone, substitué ou non ; $R_7$ représente un groupe alkyle à chaîne droite ou ramifiée ayant au moins 4 atomes de carbone, un groupe alcoxy ayant au moins 4 atomes de carbone, un groupe acylamino ayant au moins 5 atomes de carbone ou un groupe alkylsulfonamido ou arylsulfonamido ayant au moins 6 atomes de carbone ; et $Ar_2$ représente un groupe phényle ou un groupe naphtyle substitué ou non.